(19) Europäisches Patentamt
European Patent Office
Office européen des brevets

(11) Publication number: **0 275 092 B1**

(12) **EUROPEAN PATENT SPECIFICATION**

(45) Date of publication of patent specification: **03.06.92**

(51) Int. Cl.5: **C07D 487/04**, C07D 207/337, C07D 207/20, C07C 229/30, //A61K31/40,(C07D487/04, 209:00,209:00)

(21) Application number: **88100390.9**

(22) Date of filing: **13.01.88**

(54) **Process for preparing (+)-1,2-dihydro-3H-pyrrolo(1,2-a)pyrrole-1,7-dicarboxylates.**

(30) Priority: **14.01.87 US 3162**
**14.01.87 US 3104**

(43) Date of publication of application:
**20.07.88 Bulletin 88/29**

(45) Publication of the grant of the patent:
**03.06.92 Bulletin 92/23**

(84) Designated Contracting States:
**AT BE CH DE ES FR GB GR IT LI LU NL SE**

(56) References cited:
**EP-A- 0 041 711**
**EP-A- 0 114 632**
**DE-A- 2 813 373**
**EP-A- 0 053 021**
**DE-A- 2 731 662**
**US-A- 4 089 969**

(73) Proprietor: **SYNTEX (U.S.A.) INC.**
**3401 Hillview Avenue**
**Palo Alto California 94303(US)**

(72) Inventor: **Khatri, Hiralal N.**
**638 West Lilac Court**
**Louisville, Colorado 80027(US)**
Inventor: **Fleming, Michael P.**
**2692 Beech Circle**
**Longmont, Colorado 80501(US)**
Inventor: **Schloemer, George C.**
**P.O. Box 804**
**Lyons, Colorado 80540(US)**

(74) Representative: **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**W-8000 München 40(DE)**

**EP 0 275 092 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to pyrrolo[1,2-a]pyrroles, and especially to the synthesis of 1,2-dihydro-3H-pyrrolo[1,2-a]pyrrole-1,7-dicarboxylic acid and its dialkyl esters.

5-Aroyl-1,2-dihydro-3H-pyrrolo[1,2-a]pyrrole-1-carboxylic acids, also known as 5-aroyl-1,2-dihydro-3H-pyrrolizine-1-carboxylic acids, of formula I, and the

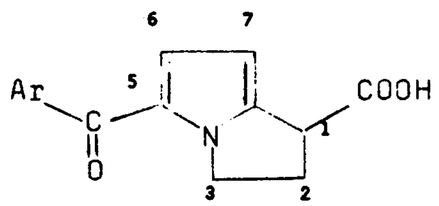

(I)

pharmacologically acceptable salts and esters thereof, are useful as analgesic, anti-inflammatory, and antipyretic agents for mammals, including man. They are also smooth muscle relaxants. Two exemplary compounds under clinical study in man are ketorolac, 5-benoyl-1,2-dihydro-3H-pyrrolo[1,2-a]pyrrole-1-carboxylic acid, (I, Ar = $C_6H_5$), and anirolac, 5-p-anisoyl-1,2-dihydro-3H-pyrrolo[1,2-a]pyrrole-1-carboxylic acid, (I, Ar = p-$CH_3O$-$C_6H_5$), both disclosed in U.S. Patent No. 4,089,969 to Muchowski et al. Other compounds, where the 5-aroyl substituents are substituted or unsubstituted benzoyl, furoyl, thenoyl, and pyrroyl, and where the 6-position on the pyrrolo-pyrrole nucleus is optionally substituted by lower alkyl or halogen, and uses thereof, are also disclosed in a series of patents assigned to Syntex (U.S.A.) Inc., beginning with U.S. Patent No. 4,089,969, and including U.S. Patents Nos. 4,087,539; 4,097,579; 4,140,698; 4,232,038; 4,344,943; 4,347,186; 4,458,081; 4,347,187; 4,454,326; 4,347,185; 4,505,927; 4,456,759; 4,353,829; 4,397,862; 4,457,941; and 4,454,151. U.S. Patents Nos. 4,511,724 and 4,536,512, assigned to Merch & Co., Inc., disclose 5-(substituted pyrrol-2-oyl)-1,2-dihydro-3H-pyrrolo-[1,2-a]pyrrole-1-carboxylic acid derivatives and 5-(1,2-dihydro-3H-pyrrolo[1,2-a]pyrrol-2-oyl)-1,2-dihydro-3H-pyrrolo[1,2-a]pyrrole-1-carboxylic acid derivatives, respectively; while U.S. Patent No. 4,533,671, also assigned to Merck & Co., Inc., discloses 5-(1,2-dihydro-3H-pyrrolo[1,2-a]pyrrol-2-oyl)-2-pyrrole-alkanoic acids and analogs.

Various methods for the preparation of these pyrrolo-pyrroles are exemplified in the patent and chemical literature, and many proceed through a common intermediate, 1,2-dihydro-3H-pyrrolo[1,2-a]-pyrrole-1,7-dicarboxylic acid, (II, R = H), or its dialkyl ester;

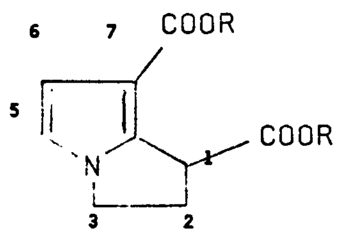

(II)

the preparation of which from dimethyl 1,3-acetone-dicarboxylate, ethanolamine, and a haloacetaldehyde is disclosed in, for example, U.S. Patent No. 4,089,969, which is incorporated herein by reference.
The reaction scheme set forth in that patent:

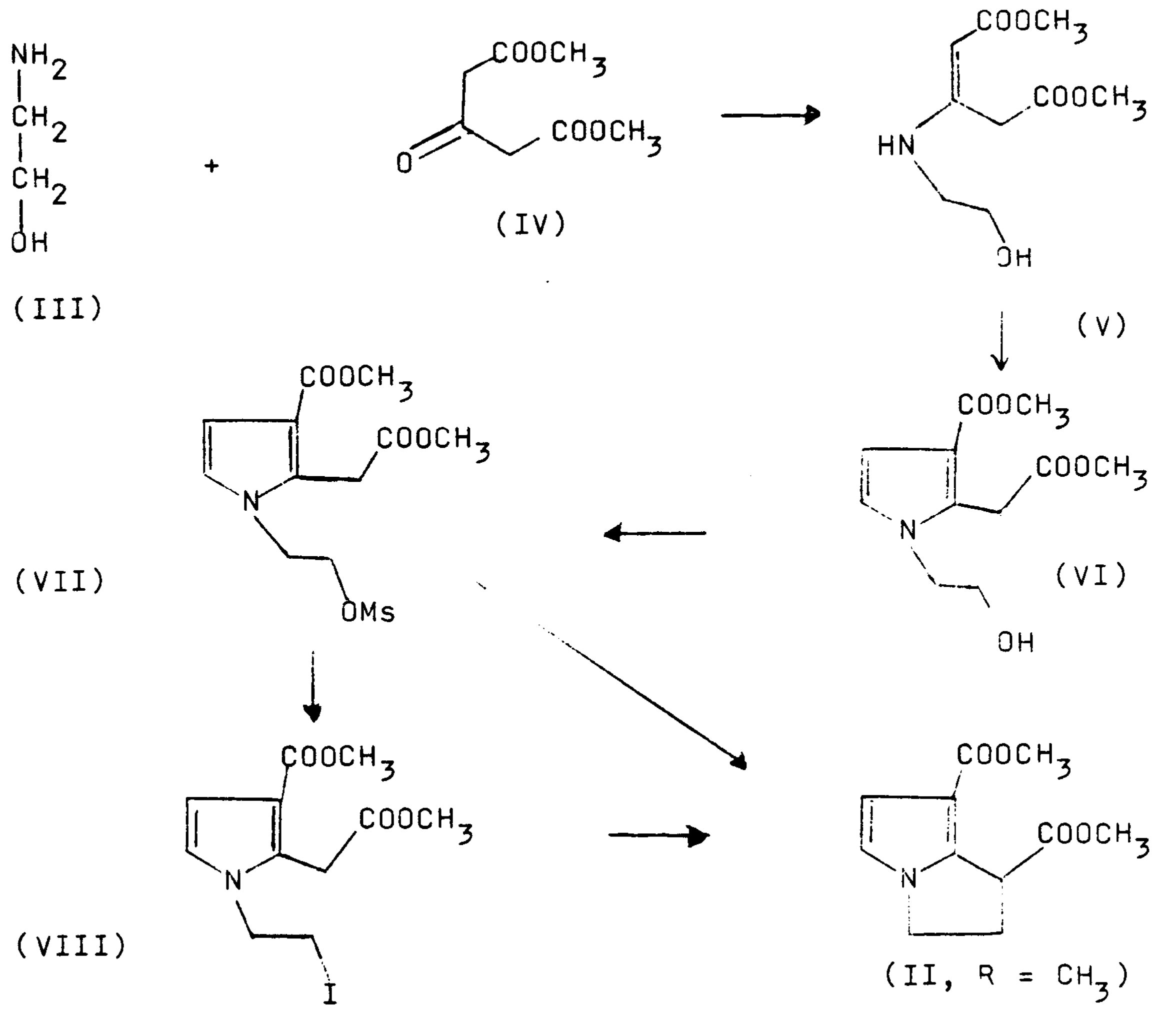

(III) (IV) (V) (VI) (VII) (VIII) (II, R = $CH_3$)

includes the reaction of equimolar amounts of ethanolamine (III) and dimethyl 1,3-acetonedicarboxylate (IV) to form a solution of the hydroxyenamine (V), which is then treated, preferably in situ, in a suitable organic solvent (aprotic solvents such as acetonitrile, dichloromethane, etc, are exemplified), under anhydrous conditions, with a 2-haloacetaldehyde at elevated temperatures to produce the N-(2-hydroxyethyl)pyrrole (VI). Compound (VI) is esterified with methanesulfonyl chloride to produce the mesylate (VII), which is optionally converted to the N-(2-iodoethyl)pyrrole (VIII) by reaction with sodium iodide. Either of compounds (VII) and (VIII) may be converted to dimethyl 1,2-dihydro-3H-pyrrolo[1,2-a]pyrrole-1,7-dicarboxylate (II, R = $CH_3$) by treatment with sodium hydride in dimethylformamide. The thus-formed dimethyl 1m2-dihydro-3H-pyrrolo[1,2-a]-pyrrole-1,7-dicarboxylate may then be selectively 7-decarboxylated and 5-aroylated, by methods such as those described in the previously-cited patents and in U.S. Patent No. 4, 496,741 to Doherty, to yield a 5-aroyl-1,2-dihydro-3H-pyrrolo-[1,2-a]pyrrole-1-carboxylic acid (I). A similar process, involving the reaction of enthanolamine, dimethyl 1,3-acetonedicarboxylate, and a halomethyl alkyl ketone, bypasses the hydroxyenamine (V) and produces the 4-alkylanalog of (VI) directly. That compound may be converted to the pyrrolo-pyrrole in the same manner as for the 4-unsubstituted compound previously described.

Various methods for the preparation of pyrroles are exemplified in the patent and chemical literature. Processes for the preparation of 5-aroyl-N-R-pyrrole-2-acetic acid, where R is H, lower alkyl, or benzyl, and analogous compounds are disclosed in U.S. Patents Nos. 3,752,826; 3,865,840; and 3,952,012 to Carson. They include, for the 4-alkyl substituted but not for the 4-unsubstituted compounds, the reaction between a lower alkylamine, a di(lower alkyl) 1,3-acetone-dicarboxylate, and a chloromethyl lower alkyl ketone, "preferably in an aqueous medium", followed by pouring into ice-cold hydrochloric acid, to produce compounds of formula (IX), where each of R, R′, and R″ is lower alkyl.

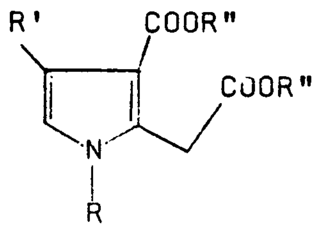

(IX)

A similar process for the 4-unsubstituted compounds (IX, R′ = H) using chloroacetaldehyde is disclosed in U.S. Patent No. 4,081,191 to Carson.

U.K. Published Applications No. 2 034 304 A, assigned to Mallinckrodt, Inc., discloses a process for the preparation of compounds of formula (IX), wherein R′ may be hydrogen or alkyl, by either (a) forming a two-phase reaction medium of an aqueous solution of the alkylamine and an inert, water-immiscible organic solvent, and adding the dicarboxylate and ketone (or aldehyde) substantially simultaneously, or (b) adding the dicarboxylate and ketone substantially simultaneously, with the ketone in excess, to an aqueous alkylamine. Preferably, dicarboxylate and ecess ketone are added to an alkylamine dispersion.

A number of patents assigned to Ethyl Corporation, including U.S. Patents Nos. 4,565,878; 4,565,879; 4,374,255; 4,388,468; 4,383,117; 4,455,433; and 4,333,878, disclose other modifications of the Carson syntheses. U.S. Patent No. 4,565,878 discloses the addition of a water-immiscible co-solvent [a halogenated hydrocarbon of good solubility for both the di(lower alkyl) 1,3-acetonedicarboxylate and the product] to the mixture of chloromethyl lower alkyl ketone, dicarboxylate, and aqueous lower alkylamine. U.S. Patent No. 4,656,789 discloses the use of an aromatic hydrocarbon as the co-solvent. U.S. Patent No. 4,374,255 discloses the addition of "a solids formation inhibiting amount of a lower alkanol" to the ketone/dicarboxylate/aqueous alkylamine mixture. The reaction is normally carried out in the presence of a co-solvent as in U.S. Patents Nos. 4,564,878 or 4,565,879. U.S. Patent No. 4,388,468 discloses a two-stage process in which (a) the ketone is added to a pre-mixed cooled solution of the alkylamine and the dicarboxylate in a suitable solvent (e.g. aromatic hydrocarbons, chlorinated hydrocarbons, water, or mixtures thereof) at a temperature less than 60°C, followed by (b) heating the reaction mixture to 70 - 100°C. U.S. Patent No. 4,383,117 discloses the use of an anhydrous lower alkylamine instead of an aqueous solution, and the use of a single-phase non-aqueous reaction medium. U.S. Patent No. 4,455,433 discloses the addition of "a yield-enhancing amount of an acid having a dissociation constant of at least $1.3 \times 10^{-5}$ at 25°C" to a ketone/dicarboxylate/(preferably anhydrous) lower alkylamine mixture, preferably in an organic solvent. U.S. Patent No. 433,878 discloses the synthesis of compounds of formula (IX) by the reaction of an enamine (X)

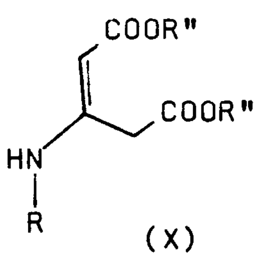

(X)

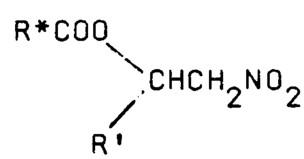

(XI)

with a 2-carboxy-1-nitroalkane (XI, R* = lower alkyl, tolyl, or benzyl). The enamine may be prepared by the reaction of a di(lower alkyl) 1,3-acetone-dicarboxylate with a lower alkylamine in "a suitable solvent, such as ethanol or methanol", followed by evaporation of the solvent and heating to dehydrate the thus-formed carbinolamine to the corresponding enamine.

Albert et al., in U.S. Patent No. 4,363,918, disclose the synthesis of compounds of formula IX where R is lower alkyl, R′ is hydrogen or lower alkyl, and R″ is H by the reaction of 1,3-acetondicarboxylic acid with $ClCH_2COR$ and a lower alkylamine, preferably by the slow addition of an aqueous solution of the lower alkylamine to an aqueous solution of the acid, followed by slow addition of the 1-chloro-2-alkanone, with both additions preferably occurring below 20°C or lower.

European Published Applications Nos. 92 487 and 105 664, assigned to Montedison S.p.A., disclose the preparation of compounds of formula IX wherein R is $CH_3$ and R″ is H, and R′ is $CH_3$ (EP92487) or H (EP105664) by the reaction of 1,3-acetonedicarboxylic acid or an alkali metal salt thereof with methylamine

and a halo(ketone or aldehyde) in aqueous solution, preferably by adding the haloketone to an aqueous mixture of the methylamine and the dicarboxylic acid.

The known processes for the preparation of pyrrolo pyrroles require high volumes of organic solvents, tend to produce tars, and are difficult to scale up to production quantities. We have now discovered a process for the preparation of pyrrolo pyrroles which uses aqueous solvents to a much greater exent than the previous known reactions and can be readily used for production scale quantities.

In a first aspect, this invention relates to the preparation of 1,2-dihydro-3*H*-pyrrolo[1,2-a]pyrrole-1,7-dicarboxylates of formula XII,

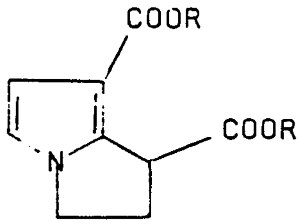

(XII)

in which

each R is independently H or lower alkyl, from di(lower alkyl) 1,3-acetonedicarboxylates.

The preparation may be represented schematically:

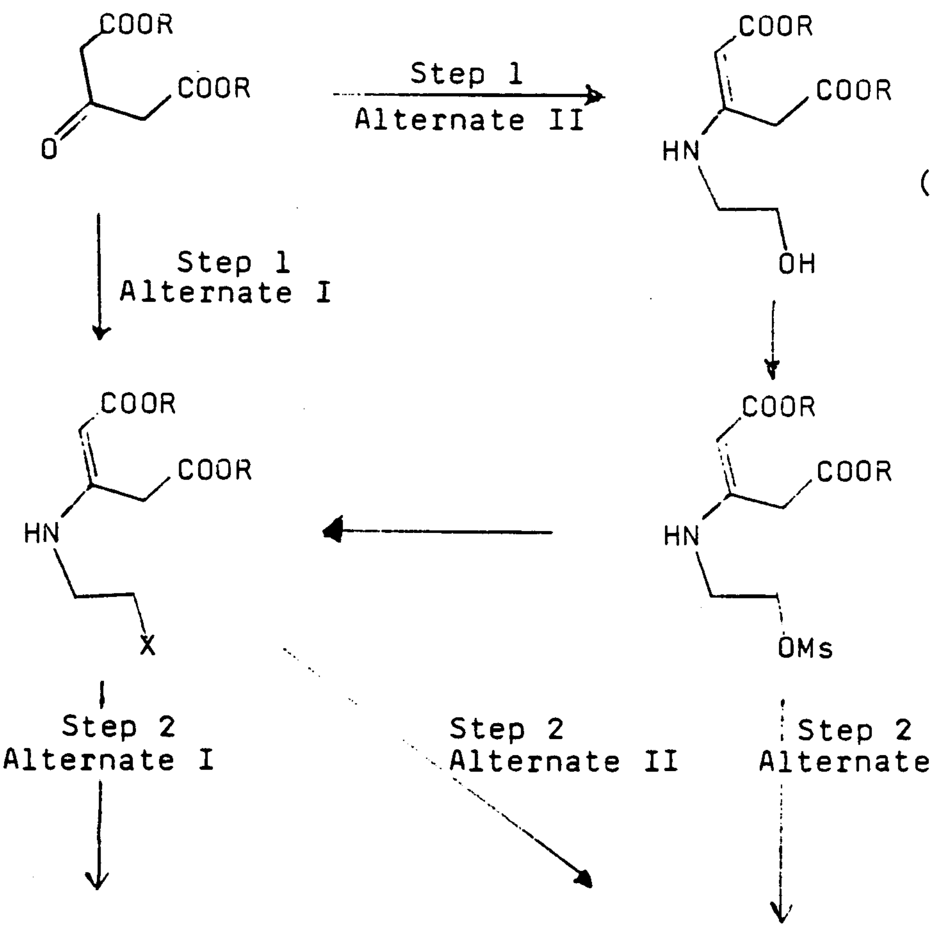


(XIV)

(XIII)

(XV)

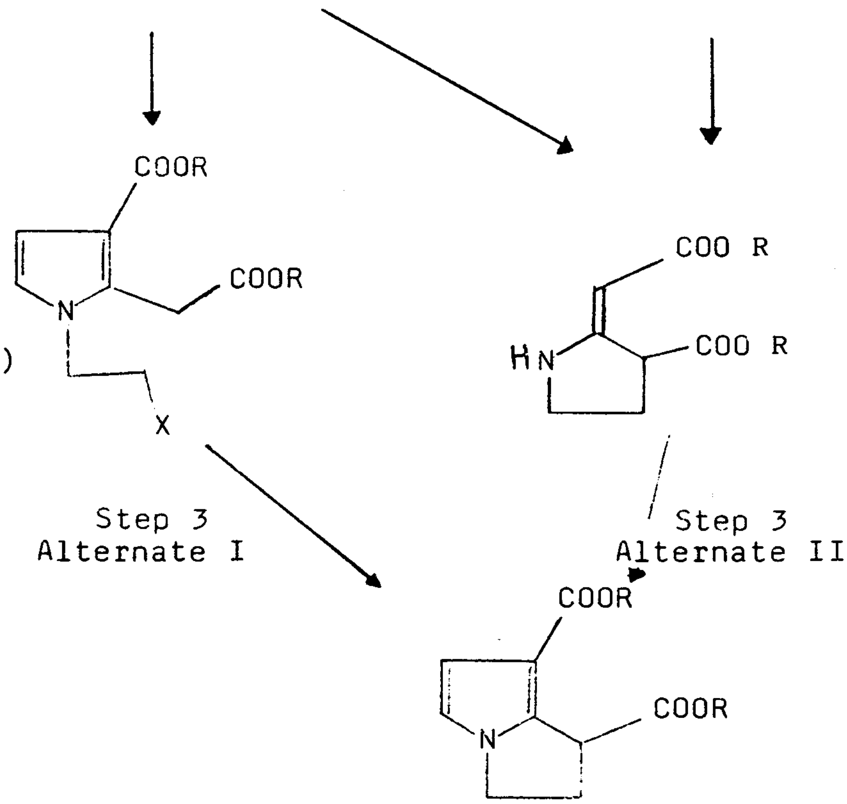


(XVI) (XVII) (XII)

in which

R is as previously defined;

Ms is mesyl; and

X is halogen.

In a second aspect, this invention relates to novel compounds of formulae (XIII), (XV), and (XVII) which are useful as intermediates in the process herein.

In another aspect, this invention relates to a process for preparing compounds of formulae (XIII) and (XVI).

In another aspect, this invention relates to a process for preparing compounds of formula (I) from compounds of formula (XII), which comprises the further steps of

(a) esterifying a compound of formula (XII) in which both R groups are hydrogen to form a compound of formula (XVIII) in which the R group at position 1 is alkyl;

(b) decarboxylating a compound of formula (XVIII) to form a compound of formula (XIX); and

(c) aroylation of a compound of formula (XIX) with an amide or morpholide to form a compound of formula (I) or (IA).

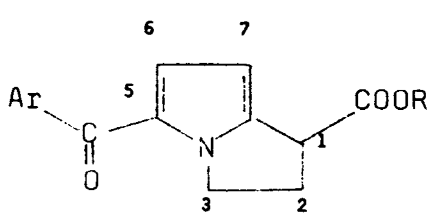

(I) or(IA)

wherein Ar is substituted or unsubstituted phenyl, 2- or 3-furyl, 2- or 3-thienyl, or 2- or 3-pyrryl and which, if substituted, can have one or more lower alkyl, lower alkoxy, or halo groups in any available position on the ring, and R is hydrogen (formula I) or alkyl of 1 to 12 carbon atoms (formula (IA).

In another aspect, this invention relates to a process for preparing compounds of formula (I) from compounds of formula (XII), which further comprises one or more of the steps:

(a) converting a compound of formula (I) to a pharmaceutically acceptable salt of a compound of formula (I); or

(b) converting a salt of a compound of formula (I) to the corresponding free compound of formula (I); or
(c) esterifying a compound of formula (I); or
(d) converting an ester of formula (IA) to the corresponding free compound of formula (I).

These steps can be illustrated by the following reaction scheme:

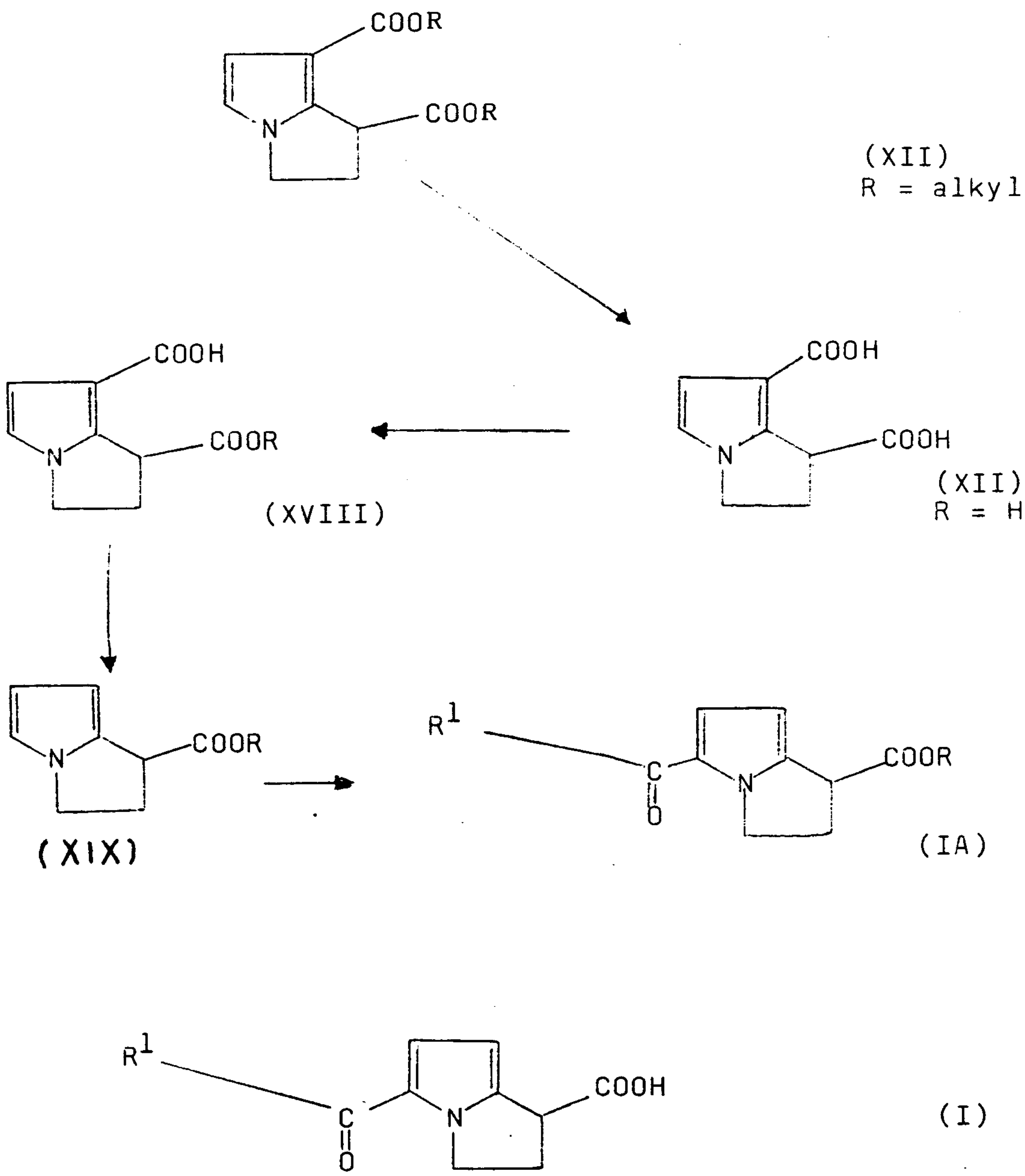

(XII) R = alkyl

(XVIII)

(XII) R = H

(XIX)

(IA)

(I)

in which R is as previously defined and R′ is substituted or unsubstituted phenyl, 2- or 3-furyl, 2- or 3-thienyl, or 2- or 3-pyrryl and which, if substituted, can have one or more lower alkyl, lower alkoxy, or halo groups in any available position on the ring.

Definitions

Unless otherwise stated, the following terms used in the specifications and claims have the meanings given below:

"lower alkyl", denoted generally by R (which may also denote hydrogen, although that is not "lower alkyl"), refers to straight, branched, or cyclic saturated hydrocarbon radicals having from one to six carbon atoms, e.g. methyl, ethyl, n-propyl, isopropyl, n-butyl, iso-butyl, t-butyl, cyclopropylmethyl, pentyl, hexyl, cyclohexyl, and the like. Preferred lower alkyls are methyl, ethyl, and n-propyl, and a particularly preferred

lower alkyl is methyl. If more than one alkyl radical is present in a given molecule, each may be independently selected from "lower alkyl" unless otherwise stated.

"lower alkoxide", "lower alkanol, "lower alkylamine", "lower alkyl ester", and similar terms refer to alkoxides, alkanols alkylamines, alkyl esters, etc. in which the (or each) alkyl radical is a "lower alkyl" as defined above.

"halogen", denoted generally by X, refers to chlorine, bromine, or iodine. Preferred halogens are chlorine and bromine.

"aprotic polar solvent" includes organic solvents which may be either water-immiscible, such as halogenated hydrocarbons, e.g. methylene chloride, chloroform, etc., or water-miscible, such a tetrahydrofuran, dimethoxyethane, bis(2-methoxyethyl) ether (also known as diglyme), dimethylformamide, N-methylpyrrolidone, dimethylsulfoxide, etc. The solvent may also contain minor proportions of aprotic nonpolar solvents such as hydrocarbons, e.g. cyclohexane, toluene, and the like, provided that the solvent properties are largely determined by the polar solvent.

"weak base" refers to the alkali metal or alkaline earth salt of a weak acid, e.g. sodium acetate, potassium bicarbonate, etc., or to a buffer mixture (such as $NaH_2PO_4/Na_2HPO_4$) giving a similar pH.

"strong base" refers to bases such as alkali metal hydroxides, lower alkoxides, hindered amines such as di(lower alkyl amines and bis(tri lower alkylsilyl) amines hydrides, and the like, which contain alkali metals including lithium, sodium, potassium, rubidium, and cesium, e.g. sodium hydroxide, potassium hydroxide, potassium methoxide, sodium methoxide, potassium ethoxide, sodium ethoxide, sodium hydride, lithium di(isopropyl)amine, lithium bis(trimethylsilyl)amine, etc.

"mesyl" or "mesylate", denoted by Ms, refers particularly to methanesulfonyl, but includes other equivalent alkyl- or arylsulfonyls, such as ethanesulfonyl, benzenesulfonyl, p-toluenesulfonyl, and the like.

"aryl" or "Ar" refers to a substituted or unsubstituted monovalent unsaturated radical, for example, phenyl, 2- or 3- furyl, 2- or 3-thienyl, or 2-or 3-pyrryl, which, if substituted, can have one or more lower alkyl, lower alkoxy, or halo groups in any available position on the ring. Phenyl and p-methoxyphenyl are preferred.

"water-miscible co-solvent" includes lower alkanols, di-(lower alkyl) ketones, tetrahydrofuran, dioxane, sulfolane, dimethylformamide, and the like. Preferred co-solvents are methanol, acetone, and similar $C_1$ or $C_2$ alkyl alcohols and ketones.

"pharmaceutically acceptable salt" refers to salts derived from inorganic bases which include sodium, potassium, lithium, ammonium, calcium, magnesium, ferrous zinc, copper, manganous, aluminum, ferric, manganic salts and the like. Particluarly preferred are the ammonium, potassium, sodium, calcium, and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-di-ethylaminoehtanol, tromethamine, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins and the like. Particularly preferred organic non-toxic bases are isopropylamine, diethylamine, ethanolamine, piperidine, tromethamine, dicyclohexylamine, choline, and caffeine.

"pharmaceutically acceptable esters" refers to alkyl esters derived from hydrocarbons of branched or straight chain having from one to twelve carbon atoms. Typical alkyl ester groups are, for example, methyl, ethyl, propyl, isopropyl, butyl, t-butyl, isoamyl, pentyl, isopentyl, hexyl, octyl, nonyl, isodecyl, 6-methyldecyl, and dodecyl.

## Starting Materials and Purification

Dimethyl 1,3-acetonedicarboxylate is commercially available (Aldrich), as is 1,3-acetonedicarboxylic acid, also known as 3-oxopentanedioic acid. Other di(lower alkyl) 1,3-acetonedicarboxylates may readily be prepared from either the dimethyl ester or, preferably, the diacid, by esterification techniques well-known to the art. However, there is no particular advantage in varying the alkyl groups, so that the dimethyl ester (R = $CH_3$) is preferred.

The starting materials and the intermediates of formulae (XIII), (XIV), (XV), and (XVI) may be isolated, if desired, using conventional techniques, including out not limited to filtration, distillation, crystallization, chromatography and the like. Such materials may be characterized using conventional means, including physical constants and spectral data.

Preparation of Compounds of Formula XIII

In Step 1, a di(lower alkyl) 3-(2-haloethylamino)-2-pentenedioate ("haloenamine") is prepared from a di-(lower alkyl) 1,3-acetonedicarboxylate, either by direct reaction with a 2-haloethylamine hydrohalide salt, or by reaction with 2-hydroxyethylamine (2-aminoethanol) followed by replacement of the hydroxy group by a halide.

In Step 1, Alternate I (which is preferred), the di(lower alkyl) 1,3-acetonedicarboxylate is treated with a 2-haloethylamine hydrohalide salt in aqueous solution. The solution preferably has a pH between 5 and 12, more preferably between 5 and 8. Typically, the pH is controlled by employing an aqueous solution of a weak base, such as sodium acetate, as the reaction solvent, but other pH control methods may be employed, if desired. The reactants may be added simultaneously or consecutively, as desired, but it is desirable that the 2-haloethylamine hydrohalide not be placed in basic solution unless that solution already contains the acetonedicarboxylate, to avoid the formation of aziridines. Typically, the 2-haloethylamine hydrohalide and acetonedicarboxylate are dissolved, either simultaneously or in the order stated, in water, and a solid weak base added to produce the reaction mixture. The reaction is preferably conducted at a temperature between 0 and 35°C, more preferably at about room temperature, i.e. between about 20 and 30°C; over a time preferably between about 15 minutes and 24 hours, more preferably between about 4 and 18 hours. The intermediate di(lower alkyl) 3-(2-haloethylamino)-2-pentenedioate ("haloenamine") typically crystallizes and may be isolated by filtration, as a mixture of the E and Z isomers. Each R is preferably methyl, X is preferably Br or Cl, and sodium acetate is preferably used to control pH.

In Step 1, Alternate II, the di(lower alkyl) 1,3-acetone-dicarboxylate is treated with 2-hydroxyethylamine (2-aminoethanol) in an aprotic polar solvent to produce the hydroxyenamine (XIV), which is then converted to the haloenamine (XIII) via the mesylenamine (XV). The conversion may stop at the mesylenamine stage, and the mesylenamine be cyclized directly (Step 2, Alternate II) if desired. Typically, the dicarboxylate is dissolved in the solvent, the 2-hydroxyethylamine is added slowly to the resulting solution, and the water formed during the reaction is removed by azeotropic distillation. While the hydroxyenamine may be formed under less stringent conditions (see, e.g., U.S. Patent No. 4,089,969), an anhydrous solution of the hydroxyenamine is required for the next reaction, and it is therefore convenient to carry out the formation of the hydroxyenamine in an aprotic medium. The resulting solution contains a mixture of the E and Z isomers of the hydroxyenamine, and may be used without purification in the next step. As in Step 1, Alternate I, a preferred R is methyl. A preferred solvent is dichloromethane.

Esterification of the hydroxyenamine (XIV) with mesyl chloride in the presence of an organic base such as a tertiary amine, optionally in the presence of an aprotic polar solvent, takes place at a temperature between about -10°C and room temperature, preferably between about 0 and 10°C. Conveniently, the tertiary amine is added to the solution from the previous reaction, which has been cooled to the appropriate temperature, and the mesyl chloride is added slowly to the resulting solution over a period between about 30 minutes and 10 hours, typically about 2 to 5 hours. The solution of the resulting mesylenamine (XV) is quenched with water, and solvent removed from the organic phase to afford a mixture of the E and Z isomers of the mesylenamine (XV), which may be used without further purification in the next reaction, though purification is preferable if the mesylenamine is to be cyclized directly rather than to be first converted to the haloenamine.

The mesylenamine (XV) is converted into the corresponding haloenamine (XIII) by reaction with an anhydrous alkali halide, preferably a bromide or iodide, e.g. sodium iodide, lithium bromide, etc., in an aprotic polar solvent at a temperature between about room temperature and the reflux temperature of the solvent e.g. between about 30 and 100°C, for 1 to 30 hours, e.g. between 5 and 20 hours, the period depending on the reagents and reaction temperature. The haloenamine (XIII) may be readily isolated by adding water to the reaction mixture, washing the organic phase, and removing the solvent to afford a mixture of the E and Z isomers of the haloenamine, which may conveniently be purified by recrystallization.

Preparation of Compounds of Formula XII

In Step 2, the haloenamine (XIII) or mesylenamine (XV) from Step 1 is cyclized with a strong base in an aprotic polar solvent.

In Step 2 (Alternate I), the haloenamine (XIII) is treated wtih a 2-haloacetaldehyde, $XCH_2CHO$, wherein X is as defined above. The process is preferably conducted in aqueous solution at a pH between 4.5 and 10, preferably between 5 and 8.5. The reaction is preferably carried out between 0 and 65°C, more preferably at about room temperature, for from about 1 to 48 hours, preferably between 6 and 24 hours. X is preferably Br, and pH control is preferably achieved by the presence of a weak base, e.g. sodium acetate or

sodium bicarbonate, in the solution. The process is preferably conducted in the presence of 10 to 50 volume percent of a water-miscible co-solvent, such as acetone.

To an aqueous solution of a weak base and the 2-haloacetaldehyde is added the haloenamine (XIII). A water-miscible co-solvent is preferably also added. The mixture is stirred for the appropriate time, and the N-(2-haloethyl)-pyrrole (XVI) is isolated by filtration. The 2-haloacetaldehyde may be obtained commercially or prepared by any desired route. In the case of the preferred 2-bromoacetaldehyde, exemplary preparative methods include the acid hydrolysis of a 2-bromoacetaldehyde di(lower alkyl) acetal, a lower alkyl 1,2-dibromoethyl ether, 1,2-dibromoethyl acetate, etc., each of which results in an aqueous solution of 2-bromoacetaldehyde to which may be added the weak base.

In Step 2, Alternate II (which is preferred), typically the haloenamine (XIII) is dissolved in the solvent, and from 1 to 2 equivalents, preferably from 1.1 to 1.5 equivalents, of the strong base added in solid form. If the base is not soluble in the solvent, it may be desirable to add a phase-transfer catalyst, such as a tetraalkylammonium halide, etc., to enhance the rate of reaction. The reaction occurs preferably between about 15 and 35 °C, more preferably at about room temperature, over from about 1 to 30 hours. The resulting cyclic enamine (XVII) may be isolated, for example by water extraction of the solution and evaporation of the solvent, and may conveniently be purified by distillation (e.g. Kugelrohr) and/or recrystallization. The E and Z isomers may be separated, if desired, but it is unnecessary to do so, as they both react in Step 3. X is preferably Br; and preferred strong bases and solvents are sodium methoxide in dichloromethane, sodium hydroxide in tetrahydrofuran or dimethylsulfoxide, sodium hydride in tetrahydrofuran, etc.

In Step 2, Alternate III (cyclization of the mesylenamine (XV)), a similar process to that described above is performed, but the quantities of base employed are generally larger, e.g. up to 6 equivalents based on the mesylenamine, and the reaction conditions more stringent (higher temperatures, e.g. up to solvent reflux temperature, and longer times). A preferred base/solvent combination is aqueous sodium hydroxide/dichloromethane, using a phase transfer catalyst.

Finally, in Step 3, Alternate I, the N-(2-haloethyl)pyrrole (XVI) is cyclized with from 1 to 2 equivalents, preferably about 1.1. to 1.5 equivalents, of a strong base in an aprotic polar solvent. The process may further include the saponification of the diester to the dicarboxylic acid (XII, R = H), which may conveniently be performed on the solution of the diester from the cyclization process.

A preferred X is Br or Cl. The strong base is prefarably a lithium nindered amine, such as lithium di-(isopropyl)amine or lithium bis(trimethylsilyl)amine; and the solvent is preferably tetrahydrofuran. Other suitable exemplary strong base/solvent combinations include sodium hydroxide in dimethylsulfoxide and potassium methoxide in methanol (although this latter tends to give an increased quantity of the N-vinylpyrrole impurity). The use of sodium hydride in dimethylformamide for the cyclization of the N-(2-iodoethyl)pyrrole is known from, e.g., U.S. Patent No. 4.089,969.

The compound of formula (XVI) is dissolved in the aprotic polar solvent, and a solution of the strong base is added slowly (i.e. over a period of from about 1 to 24 hours, e.g. about 2 to 8 hours) at a temperature of about -10 to 35 °C, e.g. at room temperature, and the resulting solution is stirred to afford a solution of the pyrrolo-pyrrole diester (XII). The reaction temperature is preferably 0 to 10 °C for X = Br, and 15 to 30 °C for X = Cl, when the strong base is lithium di(isopropyl)-amine. Compound (XII) may be recovered from the solution by removal of the solvent and purification by conventional organic chemical means, or, more usually, may be converted directly to the dicarboxylic acid (XII, R = H).

In step 3 (Alternate II), the pyrrole ring of the pyrrolo-pyrrole is formed by reaction of the cyclic enamine (XVII) with a 2-haloacetaldehyde, $XCH_2CHO$, where X is as previously defined. The process is preferably conducted in aqueous solution at a pH between 4.5 and 10, preferably between 5 and 8.5. The reaction is preferably carried out between 15 and 35 °C, more preferably at about room temperature, for from about 1 to 10 hours, preferably between 2 and 5 hours. X is preferably Br, and pH control is preferably achieved by the presence of a weak base, e.g. sodium acetate or sodium bicarbonate, in the solution. The process is preferably carried out in the presence of 10 to 50 volume percent of a water-miscible co-solvent, such as methanol.

Typically, the cyclic enamine (XVII) is added to an aqueous solution of the 2-haloacetaldehyde and a weak base. A water-miscible co-solvent is preferably also added. The mixture is stirred for the appropriate time, and the resulting compound of formula (XII) may be isolated from the reaction mixture, e.g. by evaporation of the solvent, and purified as desired. The 2-haloacetaldehyde may be obtained commercially or prepared by any desired route. In the case of the preferred 2-bromoacetaldehyde, exemplary preparative methods include the acid hydrolysis of a 2-bromacetaldehyde di(lower alkyl) acetal, a lower alkyl 1,2-dibromoethyl ether, 1,2-dibromoethyl acetate, etc., each of which results in an aqueous solution of 2-bromoacetaldehyde.

If the dicarboxylic acid, (XII, R = H), is desired, the diester may be saponified by conventional chemical means, i.e. reaction with a strong base to remove the ester groups and treatment with acid to generate the dicarboxylic acid. The diester is dissolved in an aqueous alkali metal hydroxide or carbonate solution, e.g., sodium hydroxide solution, which may also contain a water-miscible co-solvent, e.g., methanol, at a temperature between about room temperature and the solvent reflux temperature for from about 30 minutes to 24 hours, e.g. 1 to 4 hours. The cooled solution is then acidified with an aqueous strong acid, e.g. 35% hydrochloric acid, and the dicarboxylic acid precipitates and may be removed by filtration. The dicarboxylic acid may be purified by conventional means, especially conveniently by recrystallization from aqueous solution. The saponification may be performed on isolate material; but may conveniently be performed on the solution produced in the cyclization reaction described above. For example, water may be added to the solution, the aprotic polar solvent at least partially removed by distillation, and a strong base, e.g. sodium hydroxide, may be added directly to the resulting solution of the pyrrolo-pyrrole diester, the solvent further partially removed (if desired or necessary), and an aqueous strong acid, e.g. 35% hydrochloric acid, added. The dicarboxylic acid precipitates from the solution, and may be removed by filtration. The dicarboxylic acid may be purified by conventional chemical means, especially conveniently by recrystallization from aqueous solution.

The C-1 acid group of the resulting dicarboxylic acid (XII, R = H) may then be selectively esterified by treatment with a lower aliphatic alcohol. e.g., methanol ethanol, isopropanol, n-butanol, and the like in the presence of hydrogen chloride, to produce the corresponding alkyl, 1,2-dihydro-3H-pyrrolo[1,2-a]-pyrrole-1-carboxylate-7-carboxylic acid of Formula (XVIII). The reaction is conducted at a temperature of from about 0 ° C to about 50 ° C, for about 1 to about 4 hours.

Decarboxylation of the monoesterified compounds (XVIII) to the corresponding compounds of Formula (XIX) is achieved by heating (XVIII) at an elevated temperature, of the order of from about 230 ° C to about 280 ° C, for a period of time sufficient to complete the reaction. The course of the reaction can be followed by the rate of carbon dioxide evolution and thin-layer chromatography, decarboxylation being generally completed within from about 45 to about 90 minutes. The reaction product, namely, alkyl 1,2-dihydro-3H-pyrrolo[1,2-a]pyrrole-1-carboxylate and the derivatives thereof (XIX) can be purified by chromatographic techniques. Alternatively, and particularly for the decarboxylation of small batches of compound (XVIII), the reaction product (XIX) can be distilled directly from the reaction vessel.

Condensation of a compound of formula (XIX) can be accomplished in one of two ways, with an amide or with a morpholide.

Condensation of a compound of formula (XIX) with an amide of the formula

$$R^1 - \overset{O}{\overset{\|}{C}}N(CH_3)_2$$

wherein $R^1$ is one of the substituents defined above, affords the corresponding alkyl 5-aroyl-1,2-dihydro-3H-pyrrolo[1,2-a]pyrrole-1-carboxylate (IA). This reaction is conducted in an inert organic aprotic solvent and in the presence of phosphorous oxychloride, at reflux temperature for from about 1 to about 175 hours, under an inert atmosphere, followed by further reflux in the presence of sodium acetate, for from about 2 to about 10 hours. Alternatively, instead of phosphorous oxychloride, other acid chlorides such as phosgene or oxalyl chloride may be used. In the preferred embodiment, this condensation is carried out by adding a solution of compound (XIX) in a suitable solvent to a previously refluxed mixture of 1.1 to 5 molar equivalents of both the desired amide and phosphorous oxychloride in the same solvent, refluxing the reaction mixture thus obtained for from about 6 to about 72 hours under an argon atmosphere and thereafter adding thereto from about 3 to about 10 molar equivalents of sodium acetate, followed by an additional reflux period of from about 4 to about 6 hours. Adequate solvents for this reaction are the halogenated hydrocarbons such as dichloromethane, 1,2-dichloroethane, chloroform, carbon tetrachloride and the like, dimethoxyethane and tetrahydrofuran. The preferred solvent is 1,2-dichloroethane.

Represenatative of the N,N-dimethyl arylamides which can be used are: N,N-dimethyl-benzamide, N,N-dimethyl-o-toluamide, N,N-dimethyl-m-toluamide, N,N-dimethyl-p-toluamide, N,N-dimethyl-p-ethyl-benzamide, N,N-dimethyl-o-propyl-benzamide, N,N-dimethyl-m-butyl-benzamide, N,N-dimethyl-o-methoxy-benzamide, N,N-dimethyl-m-methoxy-benzamide, N,N-dimethyl-p-ethoxy-benzamide, N,N-dimethyl-p-isopropoxy-benzamide,N,N-dimethyl-o-chloro-benzamide, N,N-dimethyl-m-chloro-benzamide, N,N-dimethyl-p-chloro-benzamide, N,N-dimethyl-o-fluoro-benzamide, N,N-dimethyl-p-fluoro-benzamide, N,N-dimethyl-m-bromo-benzamide, and N,N-dimethyl-p-bromo-benzamide. These amides are known, commercially available

compounds or can be prepared in a conventional manner from the corresponding acids, i.e., by conversion into the acid chlorides followed by treatment with dimethylamine.

If an aroyl morpholide is used, the compound of formula (XIX) is treated with an aroyl morpholide of the formula

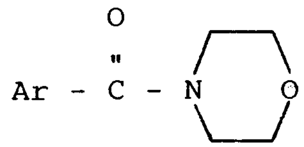

in which Ar is as defined above,
in the presence of an inorganic acid halide, such as $POCl_3$, $POBr_3$, $SO_2Cl_2$ and the like, preferably $POCl_3$. The relative amounts are not critical. Optionally an inert organic solvent such as ethane dichloride, chloroform, or carbon tetrachloride, but preferably, methylene chloride, may be included in this mixture. The presence of the solvent, however, is not particularly advantageous in all cases. The mixture is agitated, preferably stirred, for about 0.5 to 36 hours, preferably 1 to 5 hours at about 30 to 50 °C, preferably 40 to 45 °C.

A solution of the pyrrolo substrate of formula (XIX) in an inert solvent, most preferably $CH_2Cl_2$, is then added to the above mixture. Again the ratio of reactants is not critical, but it is preferable that the molar amount of the substrate be slightly less than the molar amount of the minority reactant in the prepared morpholide/halide mixture. The resulting reaction mixture is kept at about 30 to 70 °C, preferably 40 to 45 °C until the desired reaction has taken place, usually about 1 to 8 hours, most usually 1.5 to 3 hours.

The entire procedure to this point is carried out in an inert atmosphere in order to exclude water. Any anhydrous gas could be used, but nitrogen is the most convenient choice. As the reaction is scaled up, the problem of water in the ambient air becomes smaller because of less proportional available surface area. However, it has been found prudent to use nitrogen as a matter of routine.

The intermediate formed at this point cannot conveniently be isolated, but must be hydrolyzed either to the ester of formula (IA), or to the free acid of formula (I).

If it is desired to prepare a compound of formula (I), a single step procedure is preferred. In this embodiment, the reaction mixture is poured into a solution in polar solvent, preferably aqueous, of a strong base, such as a mineral hydroxide or carbonate, preferably sodium hydroxide. A large excess of the base is used. The mixture is then kept at about 30 to 100 °C, preferably 40 to 60 °C until reaction is complete.

Alternatively, if the two-step procedure is to be used, or a compound of formula (IA) is to be prepared, an amount of from about 3 to about 10 molar equivaldents of sodium acetate or other weak base may be added directly to the reaction mixture, followed by an additional reaction time of about 4 to 6 hours during which the mixture is refluxed. At the end of this period, the compound of formula (IA) is produced.

If subsequent conversion to the compound of formula (I) is desired, further hydrolysis is carried out in a conventional manner with an alkali metal hydroxide or carbonate in an aqueous or aqueous lower aliphatic alcohol (methanol, ethanol, etc.) solution. The temperature is about room temperature to reflux and the reaction time about 15 minutes to about 3 hours. Preferably, the hydrolysis is effected with aqueous methanolic potassium carbonate at reflux temperatures for about 30 minutes.

Upon alkaline hydrolysis of the alkyl ester group in a compound of formula (IA) there is obtained the corresponding free acid of formula (I). This hydrolysis is effected in a conventional manner, with an alkali metal hydroxide or alkali metal carbonate, e.g., sodium hydroxide, potassium hydroxide, sodium carbonate, potassium carbonate, and the like, in an aqueous lower aliphatic alcohol, e.g., methanol, ethanol, and the like, at a temperature of from about room temperature to reflux, for from about 15 minutes to about 2 hours, under an inert atmosphere. In the preferred embodiment, this hydrolysis is effected with aqueous methanolic potassium carbonate, at reflux temperature for about 30 minutes.

The free acids of formula (I) can be converted into other alkyl esters having from 1 to 12 carbon atoms by conventional methods, e.g., by treatment with (a) the alcohol corresponding to the desired ester in the presence of a strong mineral acid, (b) an etheral diazoalkane or (c) the desired alkyl iodide in the presence of lithium carbonate.

The salt derivatives of the compounds of formula (I) are prepared by treating the free acids with an appropriate amount of a pharmaceutically acceptable base. The reaction is conducted in water, alone or in combination with an inert, water-miscible organic solvent, at a temperature of from about 0 °C to about 100 °C, preferably at room temperature. Typical inert, water-miscible organic solvents include methanol, ethanol, isopropanol, butanol, acetone, dioxane, or tetrahydrofuran. The molar ratio of compounds of formula

(I) to base used are chosen to provide the ratio desired for any particular salt.

It should be noted that all of these methods to afford compounds of formula I are set forth in, e.g. U.S. Patent Nos. 4,089,969 and 4,353,829.

## EXAMPLES

The following Examples illustrate this invention, but are not intended to limit its scope.

Example 1: Preparation of dimethyl 3-(2-bromoethylamino)-2-pentenedioate. (Step 1 - Alternate I)

A. 2-Bromoethylamine hydrobromide (12.35 g, 60 mmol) was dissolved in water (30 mL) at room temperature (20° C) with stirring, and dimethyl 1,3-acetonedicarboxylate (10.0 g, 57 mmol) was added. After 5 to 10 minutes, solid anhydrous sodium acetate (14.35 g, 175 mmol) was added, and stirring continued. After approximately 80 minutes, precipitation of dimethyl 3-(2-bromoethylamino)-2-pentenedioate began, and the solution was stirred for 17 hours at room temperature. The thick slurry was diluted with cold water (20mL), and aged at 0 to 5° C for 30 minutes and filtered, and the precipitate washed with cold (0 to 5° C) water (50 mL) and dried to constant weight, to afford 13.9 g ( 86% yield) of dimethyl 3-(2-bromoethylamino)-2-pentenedioate as a white solid, m.p. 71 - 72° C,

NMR ($CDCl_3$):δ: 8.80 (1H, broad singlet); 4.60 (1H, singlet); 3.85 (3H, singlet); 3.77 (3H, singlet); 3.35 -3.72 (4H, broad multiplet); 3.25 (2H, singlet).

B. Substituting 2-chloroethylamine hydrochloride (7.0 g, 60 mmol) for the 2-bromoethylamine hydrobromide in the procedure of part A of this Example, there was obtained 10.8 g (80% yield) of dimethyl 3-(2-chloroethylamine)-2-pentenedioate as a white solid, m.p. 75 - 76° C,

NMR ($CDCl_3$): δ: 8.80 (1H, broad singlet); 4.60 (1H, singlet); 3.75 (3H, singlet); 3.65 (3H, singlet); 3.60 (4H, multiplet); 3.27 (2H, singlet).

C. Substituting for dimethyl 1,3-acetone-dicarboxylate in the procedure of parts A or B of this Example,

diethyl 1,3-acetonedicarboxylate
dipropyl 1,3-acetonedicarboxylate,
di(i-propyl) 1,3-acetonedicarboxylate,
di(t-butyl) 1,3-acetonedicarboxylate, or
dihexyl 1,3-acetonedicarboxylate,

one obtains, respectively,

diethyl 3-)2-bromoethylamino)-2-pentenedioate,
dipropyl 3-(2-bromoethylamino)-2-pentenedioate,
di(i-propyl) 3-(2-bromoethylamino)2-pentenedioate,
di(t-butyl) 3-(2-bromoethylamino)2-pentenedioate,
dihexyl 3-(2-bromoethylamino)2-pentenedioate,
diethyl 3-(2-chloroethylamino)-2-pentenedioate,
dipropyl 3-(2-chloroethylamino)2-pentenedioate,
di(i-propyl) 3-(2-chloroethylamino)2-pentenedioate,
di(t-butyl) 3-(2-chloroethylamino)2-pentenedioate, or
dihexyl 3-(2-chloroethylamino)-2-pentenedioate.

Example 2: Preparation of dimethyl 3-(2-bromoethylamino)-2-pentenedioate. (Step 1 - Alternate II)

A. Dimethyl 1,3-acetonedicarboxylate (196.1 g, 1.13 mol) was placed in a 3 L three-neck flask containing a stirrer bar and fitted with a thermometer, a water extractor on a reflux condenser, and an addition funnel; and the flask then purged with nitrogen. The extractor was filled with dichloromethane, and dichloromethane (800 mL) added to the flask. Ethanolamine (68.8 g, 1.13 mol) was added via the addition funnel over 10 minutes, and the mixture warmed to 30° C. The mixture was then heated under reflux for 24 hours, by which time thin-layer chromatography indicated that very little residual dicarboxylate remained and 20 mL water had collected in the extractor.

The solution of dimethyl 3-(2-hydroxyethylamino)-2-pentenedioate was cooled to 0° C, and triethylamine (235 mL, 170.6 g, 1.69 mol) was added in one portion. Methanesulfonyl chloride (168.8 g, 1.47 mol) was added dropwise via an addition funnel over 3.75 hours, with the temperature rising to 5 to 7° C. The medium-yellow slurry darkened to yellow-orange as the last 10 mL methanesulfonyl chloride was added. The mixture was stirred at 0° C for an additional 2 hours, and water (250 mL) added. The organic phase was washed with water (4 x 500 mL) and brine (250 mL), and dried overnight over

anhydrous magnesium sulfate. After filtration, the solvents were evaporated on a rotary evaporator under reduced pressure at 50°C to afford 310.0 g (93% yield) of dimethyl 3-(2-methanesulfonylamino)-2-pentendioate as a red oil. The mesylenamine may be purified at this point, if necessary or desired, and may be converted to the haloenamine by the following method or used directly in Step 2, Alternate II.

Dimethyl 3-(2-methanesulfonylamino)-2-pentenedioate (156.3 g, 529 mmol) was added to a 2 L three-neck flask, which was fitted with a mechanical stirrer, thermometer, and reflux condenser. Dichloromethane (750 mL) was added and stirred until the mesylate had completely dissolved, anhydrous lithium bromide (69.0 g, 794 mmol) was added, and the mixture stirred at 35°C for 19 hours. The mixture was cooled to 0°C and water (250 mL) added, then stirred for 5 minutes and the phases separated. The organic phase was washed with water (3 x 250 mL) and brine (150 mL), and dried over anhydrous potassium carbonate for 15 minutes. Rotary evaporation of the solvent under reduced pressure (50°C) gave 128.4 g of crude dimethyl 3-(2-bromoethylamino)-2-pentenendioate as a yellow oil, which quickly solidified to a yellow solid. The crude material was purified by extraction into boiling hexane (5 x 750 mL) and recrystallization from hexane, and the pot residues extracted and recrystallized, to give a total of 90.8 g (61.2% yield) of dimethyl 3-(2-bromoethylamino)-2-pentenedioate as white needles. An NMR spectrum in $CDCl_3$ indicated an Z/E isomer ration of 19:1, and remeasurement of the $CDCl_3$ solution after three days indicated an Z/E ratio of 4:1. The two isomers were not separated.

B. In a similar manner to that of part A of this Example, using sodium iodide in place of lithium bromide, and acetone or acetonitrile in place of dichloromethane, there was obtained in each instance crude dimethyl 3-(2-iodoethylamino)-2-pentenedioate as a light brown oil, which could be purified by conventional methods.

C. Substituting lithium chloride for lithium bromide, and using a similar procedure to that in part A of this Example, one obtains

dimethyl 3-(2-chloroethylamino)-2-pentenedioate.

D. Similarly, using other di(lower alkyl) 1,3-acetonedicarboxylates in the procedures of parts A through C of this Example, one obtains other di(lower alkyl) 3-(2-haloethylamino)-2-pentenedioates.

Example 3: Preparation of methyl N-(2-bromoethyl)-3-methoxycarbonyl-2-pyrroleacetate. (Step 2 - Alternate I)

A. (Alternate I) 2-Bromoacetaldehyde diethyl acetal (42.2 g, 214 mmol) was added to a 100 mL three-neck flask fitted with a mechanical stirrer, reflux condenser, and a thermometer. Hydrobromic acid (9 M, 4.0 mL, 36 mmol) in water (16 mL) was added, and the mixture heated under reflux for 1 hour and then cooled to room temperature to provide a solution of 2-bromoacetaldehyde.

Sodium acetate (41.0 g, 500 mmol) was added to the 2-bromoacetaldehyde solution, and stirred for 5 minutes, at which point the solution had a pH of 6. The flask was placed in a cool tap water bath and dimethyl 3-(2-bromoethylamino)-2-pentenedioate (20.0 g, 71 mmol) was added, followed by isopropanol (40 mL), and the solution was stirred at room temperature (25°C). Within about 35 minutes, all the solids had dissolved, and a precipitate started forming within 90 minutes. Stirring was continued at room temperature for 20 hours, and the solution then cooled to 0°C and stirred at that temperature for 1 hour, then filtered through a coarse frit. The precipitate was washed with ice-cold water (400 mL) and ice-cold isopropanol (150 mL) and dried, to give 15.7 g (72% yield) of crude methyl N-(2-bromoethyl)-3-methoxycarbonyl-2-pyrroleacetate as a light tan solid, m.p. 129 - 130.5°C,

NMR($CDCl_3$): $\delta$: 6.58 (2H, quartet); 4.20 (2H, triplet); 4.10 (2H, singlet); 3.75 (3H, singlet); 3.65 (3H, singlet); 3.50 (2H, triplet).

B. (Alternate I) 2-Bromoacetaldehyde diethyl acetal (20.9 g, 106 mmol) was added to a 100 mL three-neck flask fitted with a stirrer bar, reflux condenser, and a thermometer. Hydrobromic acid (48.5%, 17.7 g, 106 mmol) in water (54 mL) was added, and the mixture heated to 40°C for 3 hours and then cooled to room temperature and extracted with hexane (100 mL) to provide a solution of 2-bromoacetaldehyde.

The hydrolysis solution was added to a mechanically-stirred slurry of sodium acetate (17.8 g, 212 mmol) in water (15 mL) at 0°C over 1 hour, then stirred for a further 10 minutes. Dimethyl 3-(2-chloroethylamino)-2-pentenedioate (20.0 g, 85 mmol) was added, followed by acetone (50 mL), and the solution allowed to warm to room temperature and stirred for 20 hours. The resulting slurry was cooled to 0°C and kept at that temperature for 4 hours, then filtered through a coarse frit. The precipitate was washed with water (300 mL) and dried, to give 19.9 g (90% yield) of methyl N-(2-chloroethyl)-3-methoxycarbonyl-2-pyrroleacetate as white crystals, m.p. 110 - 111°C,

NMR ($CDCl_3$): $\delta$: 6.65 (2H, quartet); 4.25 (2H, triplet); 4.18 (2H, singlet); 3.82 (3H, singlet); 3.75 (2H, triplet); 3.73 (3H, singlet).

C. (Alternate I) Substituting 2-chloroacetaldehyde diethyl acetal for 2- bromoacetaldehyde diethyl acetal, and using a similar procedure to that in parts A or B of this Example, one obtains

methyl N-(2-bromoethyl)-3-methoxycarbonyl-2-pyrroleacetate, or

methyl N-(2-chloroethyl)-3-methoxycarbonyl-2-pyrroleacetate.

D. (Alternate I) Similarly, substituting other di(lower alkyl) 3-(2-haloethylamino)-2-pentenedioates, such as dimethyl 3-(2-iodoethylamino)-2-pentenedioate, diethyl 3-(2-bromoethylamino)-2-pentenedioate, etc., for the equivalent materials in parts A through C of this Example, one obtains other alkyl N-(2-haloethyl)-3-alkoxycarbonyl-2-pyrroleacetates, such as methyl N-(2-iodoethyl)-3-methoxycarbonyl-2-pyrroleacetate, ethyl N-(2-bromethyl)-3-ethoxycarbonyl-2-pyrroleacetate, etc.

Example 4: Preparation of methyl 3-methoxycarbonyl-2-pyrrolidinylidenecarboxylate. (Step 2 - Alternate II)

A. (Alternate II) Dimethyl 3-(2-bromoethylamino)-2-pentenedioate (5.00 g, 17.8mmol) was added to a 100 mL flask containing a stirrer bar. Dichloromethane (50 mL) was then added, and the mixture stirred until complete dissolution occurred. Sodium methoxide (1.92 g, 35.5 mmol) was added in portion over 18 minutes, with some heat evolution. The reaction mixture was stirred at room temperature for 23 hours, then extracted with water (4 x 50 mL) and brine (50 mL) and dried over anhydrous magnesium sulfate. Rotary evaporation of the solvents gave 2.27 g of a pale yellow solid, which was separated by preparative thin-layer chromatography into three components. The major product was recrystallised from hexane to afford crystalline methyl (Z)-3-methoxycarbonyl-2-pyrrolidinylidenecarboxylate, m.p. 84 - 85 °C,

NMR ($CDCl_3$): δ: 7.8 - 8.0 (1H, broad singlet); 4.70 (1H, doublet, J = 0.67 Hz); 3.75 (3H, singlet); 3.64 (3H, singlet); 3.5 0 3.8 (3H, multiplet); 2.1 - 2.5 (2H, multiplet).

A minor product was isolated as an oil, methyl (E)-3-methoxycarbonyl-2-pyrrolidinylidenecarboxylate, NMR ($CDCl_3$): δ: 7.8 - 8.0 (1H, broad singlet); 4.64 (1H, singlet); 4.08 (1H, doublet, J = 11 Hz); 3.77 (3H, singlet); 3.65 (3H, singlet); 3.55 (1H, doublet, J = 11 Hz); 3.5 - 3.9 (1H, multiplet); 2.0 - 2.4 (2H, multiplet). A very minor product was also isolated as an oil, dimethyl 3-(1-aziridinyl)-2-pentenedioate.

B. (Alternate II) To a solution of dimethyl 3-(2-chloromethylamino)-2-pentenedioate (4.71 g, 20 mmol) in dichloromethane (50 mL) was added sodium methoxide (1.62 g, 30 mmol) in four equal portions over 45 minutes at room temperature. The reaction mixture, which had developed some yellow color and precipitate, was stirred for 27 hours, then worked up as described in part A of this Example to yield 2.8 g of a yellow oil, which was determined by preparative thin-layer chromatography and NMR to contain methyl (Z)- and (E)- 3- methoxycarbonyl-2-pyrrolidinylidenecarboxylates.

C. (Alternate II) To a solution of dimethyl 3-(2-iodoethylamino)-2-pentenedioate (3.3 g, 10 mmol) in tetrahydrofuran (distilled, 20 mL) was added tetrabutylammonium bromide (0.32 g, 1 mmol) and sodium hydroxide beads (0.41 g, 10.3 mmol), and the resulting mixture stirred at room temperature for 5.5 hours, during which it developed a lime color. Dichloromethane (30 mL) and water (20 mL) were added, and the mixture stirred for 5 minutes, giving a light brown aqueous layer and a pale yellow lower organic layer. The organic layer was separated, and the organic layer washed with dichloromethane (2 x 20 mL). The washings were combined with the organic layer, and the combined organic phase washed with water (2 x 20 mL) and brine (20 mL). Removal of the solvents by rotary evaporation gave 1.8 g (90% yield) of a light brown oil, which solidified on standing. Thin-layer chromatography and NMR spectra indicated major and minor proportions of methyl (Z)- and (E)-3-methoxycarbonyl-2-pyrrolidinylidene-carboxylates, with a very minor proportion of the elimination product dimethyl 3-(vinylamino)-2-pentenedioate.

D. (Alternate II) Similarly, using other di(lower alkyl) 3-(2-haloethylamino)-2-pentenedioates and, optionally, other strong bases and/or aprotic polar solvents, in the procedures of parts A through C of this Example, one obtains other (lower alkyl) 3-(lower alkoxy)carbonyl-2-pyrrolidinylidenecarboxylates.

E. (Alternate III) To a nitrogen-purged three-neck 1 L flask containing a spin bar and fitted with a reflux condenser were added sodium hydroxide (32.00 g, 800 mmol) and water (32.0 mL), and these were stirred until complete solution was obtained. Dichloromethane (640 mL) was then added, followed by dimethyl 3-(2-methanesulfonyl-ethylamino)-2-pentenedioate (40.12 g, 136 mmol) and tetrabutylammonium bromide (6.57 g, 20 mmol). The reaction mixture was heated at reflux for 7 hours, at which time thin-layer chromatography revealed the presence of a small quantity of starting mesylenamine. The reaction mixture was therefore stirred for a further 14 hours at room temperature, at which time thin-layer chromatography showed no mesylenamine remaining, then washed with water (3 x 200 mL) and brine (200 mL), and dried over anhydrous magnesium sulfate to afford a yellow solution. Rotary evaporation of the solvent under reduced pressure gave a red, mobile liquid, which was distilled (Kugelrohr, 0.3 mm Hg, 50 to 55 °C) to afford 19.74 g (72.9% yield, distilled) of a mixture of methyl (Z)- and (E)-3-methoxycarbonyl-2-pyrrolidinylidene-carboxylates as a clear, colorless oil.

F. (Alternate III) Similarly, using other di(lower alkyl) 3-(2-mesylethylamino)-2-pentenedioates and, optionally, other strong bases and/or aprotic polar solvents, in the procedure of part E of this Example, one obtains other (lower alkyl) 3-(lower alkoxy)carbonyl-2-pyrrolidinylidenecarboxylates.

Example 5: Preparation of dimethyl1,2-dihydro-3H-pyrrolo[1,2-a]pyrrole-1,7-dicarboxylate and 1,2-dihydro-3Hpyrrolo[1,2-a]pyrrole-1,7-dicarboxylic acid. (Step 3)

A. (Alternate I) Under nitrogen, n-butyllithium 1.3 m in hexane, 75 mL, 98 mmol) was added slowly at -5 to 0 °C, with stirring, to a solution of di(isopropyl)amine (13.8 mL, 10.0 g, 98 mmol) in tetrahydrofuran (dry, 50 mL). The resulting solution was transferred to an addition funnel and added, under nitrogen at 0 to 5 °C, to a stirred slurry of methyl N-(2-bromoethyl)-3-methoxycarbonyl-2-pyrroleacetate (20.0 g, 66 mmol) in tetrahydrofuran (dry, 100 mL). There was a slight temperature rise, and complete dissolution occurred after addition of about two-thirds of the lithium di(isopropyl)amine solution. The resulting solution was stirred for 2 hours while warming to 10 °C, then diluted with water (100 mL) with slight heat evolution. The solvents were stripped by atmospheric distillation: 240 mL collected, with a pot temperature of 80 °C, to afford a solution of dimethyl 1,2-dihydro-3H-pyrrolo-[1,2-a]pyrrole-1,7-dicarboxylate. The diester may be isolated at this point by quenching with water, extraction into an organic solvent such as ethyl acetate, and evaporation of the solvent; and purified by conventional methods. However, it is also convenient to saponify the diester to the dicarboxylic acid without isolation from the solution.

The pot solution from the previous paragraph was cooled to 50 °C and sodium hydroxide (6.0 g, 150 mmol) added; then methanol was removed by atmospheric distillation to a pot temperature of 97 °C. The pot solution was cooled to 5 °C and acidified with hydrochloric acid (12 M, 18 mL, 216 mmol), resulting in a temperature rise to 15 °C. The resulting mixture was cooled to 5 °C and filtered, and the precipitate washed with cold water (50 mL) and dried, to give 11,4 g of crude 1,2-dihydro-3H-pyrrolo[1,2-a]pyrrole-[1,2-a]pyrrole-1,7-dicarboxylic acid (and 7.0% N-vinyl-3-carboxy-2-pyrroleacetic acid).

B. (Alternate I) n-Butyllithium (2.6 M in hexane, 295.5 mL, 0.77 mol) was added dropwise at 0 to 3 °C, with stirring, to a solution of di(isopropyl)amine (109.3 mL, 78.9 g, 0.78 mol) in tetrahydrofuran (freshly distilled, 200 mL). The resulting solution was transferred to an addition funnel and added dropwise at room temperature to a stirred solution of methyl N-(2-chloroethyl)-3-methoxycarbonyl-2-pyrroleacetate (150.0 g, 0.58 mol) in tetrahydrofuran (dry, 750 mL). The addition took 4 hours, and the temperature was maintained in the range of 23 to 27 °C. The resulting solution was stirred for 16 hours, then diluted with water (500 mL) over 10 minutes with slight heat evolution. The solvents were stripped by atmospheric distillation to a pot temperature of 75 °C. The pot solution was cooled to 50 °C and soidum hydroxide (53.0 g, 1.33 mol) added; then methanol was removed by atmospheric distillation to a pot temperature of 95 °C; with a total of 1370 mL of solvents collected. The pot solution was coolted to -3 to 0 °C and acidified with hydrochloric acid (12 M, 180 mL, 216 mmol), resulting in a temperature rise to 18 °C. The resulting mixture was cooled to -3 to 0 °C, aged for 30 minutes at that temperature, and filtered, and the precipitate washed with ice-cold water (300 mL) and dried, to give 107.8 g of crude 1,2-dihydro-3H-pyrrolo[1,2-a]pyrrolo-1,7-dicarboxylic acid. Assay of the crude material indicated that it contained 96.6% 1,2-dihydro-3H-pyrrolo-[1,2-a]pyrrole-1,7-dicarboxylic acid.

C. (Alternate I) Similarly, substituting for the methylN-(2-bromoethyl)-3-methoxycarbonyl-2-pyrroleacetate or methyl N-(2-chloroethyl)-3-methoxy-carbonyl-2-pyrroleacetate of parts A and B of this Example,

methyl N-(2-iodoethyl)-3-methoxycarbonyl-2-pyrroleacetate,

ethyl N-(2-chloroethyl)-3-ethoxycarbonyl-2-pyrroleacetate,

and similar (lower alkyl) N-(2-haloethyl)-3-(lower alkoxy)carbonyl-2-pyrroleacetates, one obtains

dimethyl 1,2-dihydro-3H-pyrrolo[1,2-a]pyrrole-1,7-dicarboxylate or 1,2-dihydro-3-H-pyrrolo[1,2-a]-pyrrole-1,7-dicarboxylic acid,

diethyl 1,2-dihydro-3H-pyrrolo[1,2-a]pyrrole-1,7-dicarboxylate or 1,2-dihydro-3H-pyrrolo[1,2-a]pyrrole-1,7-dicarboxylic acid,

and similar di(lower alkyl) 1,2-dihydro-3H-pyrrolo[1,2-a]-pyrrole-1,7-dicarboxylates or 1,2-dihydro-3H-pyrrolo-[1,2-a]pyrrole-1,7-dicarboxylic acids.

D. (Alternate II) 2-Bromoacetaldehyde dimethyl acetal (1.70 g, 10 mmol), hydrobromic acid (48.5%, 0.25 mL, 2 mmol), and water (2.5 mL) were heated at reflux for 75 minutes, to provide a solution of 2-bromoacetaldehyde. The solution was cooled to room temperature, and sodium acetate (0.50 g) added. The resulting solution was added over 3 hours to a stirred mixture of methyl 3-methoxycarbonyl-2-pyrrolidinylidenecarboxylate (1.00 g, 5 mmol), sodium acetate (3.10 g), and water (2 mL) in methanol (10 mL). The mixture was stirred an additional hour, and most of the solvents then removed by vacuum

evaporation. The residue was dissolved in dichloromethane (100 mL), and the solution washed with aqueous sodium bicarbonate (2 x 25 mL) and dried over anhydrous magnesium sulfate. Removal of the solvent by evaporation gave 1.26 g of dimethyl 1,2-dihydro-3H-pyrrolo[1,2-a]pyrrole-1,7-dicarboxylate.

E. (Alternate II) Substituting 2-chloroacetaldehyde diethyl acetal for 2-bromoacetaldehyde diethyl acetal, and using a similar procedure to that in part D of this Example, one obtains

dimethyl 1,2-dihydro-3H-pyrrolo[1,2-a]pyrrole-1,7-dicarboxylate.

F. (Alternate II) Similarly, substituting other (lower alkyl) 3-(lower alkoxy)carbonyl-2-pyrrolidinylidenecarboxylates, such as ethyl 3-ethoxycarbonyl-2-pyrrolidinylidenecarboxylate, n-propyl 3-propoxycarbonyl12-pyrrolidinylidenecarboxylate, etc., for the equivalent materials in parts D and E of this Example, one obtains other di(lower alkyl) 1,2-dihydro-3H-pyrrolo[1,2-a]-pyrrole-1,7-dicarboxylates, such as diethyl 1,2-dihydro-3H-pyrrolo[1,2-a]-pyrrole-1,7-dicarboxylate, di(n-propyl) 1,2-dihydro-3H-pyrrolo[1,2-a]pyrrole-1,7-dicarboxylate, etc.

Example 6: Preparation of 1,2-dihydro-3H-pyrrolo[1,2-a]-pyrrole-1,7-dicarboxylic acid.

A. Dimethyl 1,2-dihydro-3H-pyrrolo[1,2-a]pyrrole-1,7-dicarboxylate (1.26 g, 5 mmol) and sodium hydroxide (1.00 g, 25 mmol) were heated in water (10 mL) at reflux for 1 hour. The resulting solution was cooled to 0 ° C and acidified with hydrochloric acid (12 M) to pH 1. 1,2-Dihydro-3H-pyrrolo[1,2-a]pyrrole-1,7-dicarboxylic acid (0.70 g, 71.4% yield) was collected by filtration and dried.

B. Similarly, substituting for the dimethyl 1,2-dihydro-3H-pyrrole-1,7-dicarboxylate of part A of this Example,

diethyl 1,2-dihydro-H-pyrrolo[1,2-a]pyrrole-1,7-dicarboxylate,

and similar di(lower alkyl) 1,2-dihydro-3H-pyrrolo[1,2-a]pyrrole-1,7-dicarboxylate, one obtains

1,2-dihydro-3H-pyrrolo[1,2-a]pyrrole-1,7-dicarboxylic acid.

Example 7: Preparation of 5-aroyl-1,2-dihydro-3H-pyrrolo[1,2-a]pyrrole-1-carboxylic acids

A. A solution of 1.34 g of 1,2-dihydro-3H-pyrrolo[1,2-a]pyrrole-1,7-dicarboxylic acid in 50 ml of isopropanol, cooled in an ice bath is saturated with gaseous hydrogen chloride, maintaining the temperature of the reaction mixture below 50 ° C. The ice bath is then removed and the reaction mixture is stirred for 1.5 hours at room temperature, and evaporated to dryness under reduced pressure; 10 ml of benzene is added to the residue and the solution is evaporated under vacuum once again, repeating this process a total of three times to completely remove the excess hydrogen chloride, thus obtaining 1.58 g (96%) of isopropyl 1,2-dihydro-3H-pyrrolo[1,2-a]pyrrole-1-carboxylate-7-carboxylic acid, which upon crystallizatin from methanol-ethyl acetate has a melting point of 144-145 ° C.

B. 1.054 g of isopropyl 1,2-dihydro-3H-pyrrolo[1,2-a]pyrrole-1-carboxylate-7-carboxylate acid is heated to 240-250 ° C in a dry 10 ml round bottomed flask, distilling directly the reaction product from the reaction vessel. In this manner there is obtained 745 mg (87%) of isopropyl 1,2-dihydro-3H-pyrrolo[1,2-a]pyrrole-1-carboxylate, a pale yellow oil, having the following physical constants:

U.V. $\lambda_{max}^{MeOH}$ 215 nm ($\varepsilon$ 6020); I.R. $\nu_{max}^{CHCl_3}$ 1725 $cm^{-1}$; N.M.R. $\delta_{TMS}^{CDCl_3}$ 1.22 (d. J= 7 Hz, 6H), 2.40-2.90 (m, 2H), 3.60-4.20 (m, 2H), 4.65-5.2 (m, 1H), 5.73-5.92 (m, 1H), 6.10 (t, J= 3 Hz, 1H), 6.43-6.53 ppm (m, 1H).

C. A 100 ml 3-necked round bottomed flask equipped with a condenser, nitrogen inlet tube and a gas bubbler is charged with 5.0 g of isopropyl 1,2-dihydro-3H-pyrrolo[1,2-a]pyrrole-1-carboxylate-7-carboxylic acid. The apparatus is thoroughly flushed with nitrogen and then the nitrogen flow is stopped. The apparatus is immersed in an oil bath heated at 270 ° C and the reaction is followed by the rate of carbon dioxide evolution (gas bubbler) and by t.l.c. on silica gel, using benzene:dioxane:acetic acid (90:10:1) as developing solvent. After 45 minutes the reaction is almost complete. After one hour, the vessel is removed from the oil bath and the contents of the reaction flask are transferred to a round bottomed flask with 500 ml of acetone. The solvent is removed under reduced pressure, and the residue is purified by column chromatography on 100 g of silica gel. The fractions eluted with hexane:benzene (70:30) and

hexane:benzene (50:50) afford 2.77 g (68%) of isopropyl 1,2-dihydro-3H-pyrrolo[1,2-a]-pyrrole-1-carboxylate, an oil.

D. A solution of 179 mg of N,N-dimethyl-p-toluamide and 0.11 ml of phosphorous oxychloride in 2 ml of 1,2-dichloroethane is refluxed for 30 minutes. To this solution is added a solution of 193 mg of isopropyl 1,2-dihydro-3H-pyrrolo[1,2-a]pyrrole-1-carboxylate in 2 ml of 1,2-dichloroethane. The reaction mixture is refluxed under an argon atmosphere for 8 hours, treated with 405 mg of sodium acetate and refluxed for a further 5 hours. The resultant mixture is then evaporated to dryness and the residue is chromatographed on 12 g of silica gel, eluting with hexane:ethyl acetate (3:1), thus obtaining 208 mg (66%) of isopropyl 5-p-toluoyl-1,2-dihydro-3H-pyrrolo-[1,2-a]pyrrole-1-carboxylate, an oil, having the following physical constants:

U.V. $\lambda_{max}^{MeOH}$ 256, 312 nm ($\varepsilon$ 8700, 19500); I.R. $\nu_{max}^{film}$ 1735, 1620, 1605 $cm^{-1}$; N.M.R. $\delta_{TMS}^{CDCl3}$ 1.23 (d, J = 7 Hz, 6H), 2.38 (s, 3H), 2.5-3.0 (m, 2H), 3.75-4.10 (m, 1H), 4.2-4.60 (m, 2H), 4.85-5.20 (m, 1H), 5.95 (d, J = 4 Hz, 1H), 6.70 (d, J = 4 Hz, 1H), 7.10 (d, J = 8 Hz, 2H), 7.60 ppm (d, J = 8 Hz, 2H).

E. A solution of 336 mg of isopropyl 5-p-toluoyl-1,2-dihydro-3H-pyrrolo[1,2-a]pyrrole-1-carboxylate in 10 ml of methanol is treated with a solution of 690 mg of potassium carbonate in 5 ml of water. The reaction mixture is refluxed under nitrogen atmosphere for 30 minutes, cooled, and evaporated to dryness. The residue is taken up in 10 ml of 10% aqueous hydrochloric acid and 50 ml of water and the resultant mixture extracted with ethyl acetate (2 x 50 ml). The combined extracts are dried over magnesium sulfate and evaporated to dryness under reduced pressure. Crystallization of the residue from ethyl acetate-hexane affords 238 mg (89%) of 5-p-toluoyl-1,2-dihydro-3H-pyrrolo[1,2-a]pyrrole-1-carboxylic acid, m.p. 182-183 ° C.

F. A solution of 250 mg of isopropyl 5-p-toluoyl-1,2-dihydro-3H-pyrrolo[1,2-a]pyrrole-1-carboxylate in 8 ml of methanol is treated under an atmosphere of nitrogen, with a solution of 200 mg of sodium hydroxide in 1 ml of water, maintaining the reaction mixture at room temperature for 1.5 hours. The methanol is then removed under reduced pressure and the basic solution which remains is diluted with 5 ml of water and extracted with ether to remove any unsaponifiable product. The aqueous solution is acidified with 10% hydrochloric acid and extracted three times with ethyl acetate. The combined extracts are dried and evaporated to dryness under reduced pressure, and the residue crystallized from ethyl acetate-hexane, to give 5-p-toluoyl-1,2-dihydro-3H-pyrrolo[1,2-a]pyrrole-1-carboxylic acid.

G. By following the above methods using 1.1 to 5 molar equivalents of N,N-dimethyl-benzamide in place of N,N-dimethyl-p-toluamide, and monitoring the course of the reaction by t.l.c., there is obtained:

isopropyl-5-benzoyl-1,2-dihydro-3H-pyrrolo[1,2-a]-pyrrole-1-carboxylate, a light yellow oil, having the following physical constants:

U.V. $\lambda_{max}^{MeOH}$ 245, 311 nm ($\varepsilon$ 7230, 17800); I.R. $\nu_{max}^{CHCl3}$ 1735, 1620 $cm^{-1}$; N.M.R. $\delta_{TMS}^{CDCl3}$ 1.24 [d, 6H, $(CH_3)_2CH$], 2.50-3.13 (m, 2H; H-2), 3.97 (dd, 1H, H-1), 4.18-4.70 (m, 2H, H-3), 5.00 [sept., 1H, $(CH_3)_2CH$], 6.00 (d, 1H, H-7), 6.86 (d, 1H, H-6), 7.10-7.90 ppm (m, 5H, phenyl protons); M.S.: m/e 297 (M+).

Upon hydrolysis of the isopropyl ester group, in accordance with the above methods, there is obtained: 5-benzoyl-1,2-dihydro-3H-pyrrolo[1,2-a]pyrrole-1-carboxylic acid, m.p. 160-161 ° C.

H. Benzomorpholide (1.45 g, 7.90 mmole), was placed in a 25 ml round bottom flask. $POCl_3$ (1.25 ml, 13.4 mmole) was added. This was stirred and heated in a 40 ° oil bath for 2.5 hours. A solution of methyl 1,2-dihydro-3H-pyrrolo[1,2-a]pyrrole-1-carboxylate (1.00 g, 6.10 mmole) in 3.4 ml $CH_2Cl_2$ was added and heating was continued for 2 hours, at this time, thin layer chromatography (TLC) of the reaction mixture showed reaction to be complete. The reaction mixture was added carefully to a solution of NaOH (3.09 g, 77.3 mmole) in 10 ml $H_2O$, allowing the $CH_2Cl_2$ to boil off, and the mixture heated to 50 ° . More NaOH (1.30 g, 32.5 mmole) was added. TLC 15 minutes later showed complete hydrolysis. The mixture was cooled and extracted with 2 x 10 ml $CH_2Cl_2$. The aqueous layer was acidified by the addition of 3.30 ml (39.6 mmole) conc. HCl. A milk formed. This was extracted with 5 x 10 ml $CH_2Cl_2$. Extract numbers 1-4 were dried ($Na_2SO_4$) and charcoaled (Darco 660), then evaporated to a tan solid, which was a mixture of all benzoyl isomers. This solid was dissolved in 10 ml 2-propanol and hexane (10 ml) was added. A crystalline solid formed slowly. The first crop had a weight of 0.90 g (58.5%) and was pure (by TLC) 5-benzoyl-1,2-dihydro-3H-pyrrolo[1,2-a]pyrrole-1-carboxylic acid, m.p. 160-161 ° C.

I. A solution of 200 mg of 5-benzoyl-1,2-dihydro-3H-pyrrolo[1,2-a]pyrrole-1-carboxylic acid in 5 ml of dichloromethane is treated with an excess of ethereal diazomethane, and the reaction mixture is maintained at room temperature for 30 minutes. The solvents and excess reagent are eliminated under reduced pressure and the residue crystallized from ethyl acetate-methanol, to yield methyl 5-benzoyl-1,2-dihydro-3H-pyrrolo[1,2-a]pyrrole-1-carboxylate.

J. A solution of 300 mg of 5-p-toluoyl-1,2-dihydro-3H-pyrrolo[1.2-a]pyrrole-1-carboxylic acid in 5 ml of isoamyl alcohol is saturated with hydrogen chloride. After 24 hours, the excess alcohol is distilled off in vacuo and the residue purified by chromatography on alumina, to yield isoamyl 5-p-toluoyl-1,2-dihydro-3H-pyrrolo[1,2-a]pyrrole-1-carboxylate.

K. To a solution of 300 mg of 5-p-toluoyl-1,2-dihydro-3H-pyrrolo[1,2-a]pyrrole-1-carboxylic acid in 5 ml of methanol is added 1 molar equivalent of sodium hydroxide, in the form of a 0.1N solution. The solvent is then evaporated under reduced pressure and the residue taken up in 2 ml of methanol, followed by precipitation with ether, to yield crude sodium 5-p-toluoyl-1,2-dihydro-3H-pyrrolo[1,2-a]pyrrole-1-carboxylate which can be crystallized from ethyl acetate-hexane.

L. To a solution of 175 mg of 5-p-toluoyl-1,2-dihydro-3H-pyrrolo[1,2-a]pyrroel-1-carboxylic acid in 5 ml of methanol is added 1 molar equivalent of potassium hydroxide, in the form of a 0.1N solution, thus yielding a solution containing potassium 5-p-toluoyl-1,2-dihydro-3H-pyrrolo[1,2-a]pyrrole-1-carboxylate. A solution of 40 mg of calcium carbonate dissolved in the minimum amount of 1N hydrochloric acid necessary to effect solution of the calcium carbonate, is buffered with 100 mg of solid ammonium chloride, followed by the further addition of 5 ml of water. The thus obtained buffered calcium solution is then added to the solution of potassium 5-p-toluoyl-1,2-dihydro-3H-pyrrolo[1,2-a]-pyrrole-1-carboxylate and the precipitate which forms is collected by filtration, washed with water and air dried, to yield calcium 5-p-toluoyl-1,2-dihydro-3H-pyrrolo-[1,2-a]-pyrrole-1-carboxylate.

M. A solution of 200 mg of 5-p-toluoyl-1,2-dihydro-3H-pyrrolo[1,2-a]pyrrole-1-carboxylic acid in 15 ml of hot benzene is treated with 60 mg of ispropylamine. The solution is allowed to cool to room temperature and the product filtered off, washed with ether and dried to yield the isopropylamine salt of 5-p-toluoyl-1,2-dihydro-3H-pyrrolo[1,2-a]pyrrole-1-carboxylic acid.

N. 100 mg of sodium 5-benzoyl-1,2-dihydro-3H-pyrrolo[1,2-a]pyrrole-1-carboxylate is dissolved in 50 ml of water. 10 ml of concentrated hydrochloric acid are added with stirring at room temperature. The

aqueous solution is then extracted with 2 x 50 ml portions of ethyl acetate, the organic extracts combined, and dried and evaporated. The product, 5-benzoyl-1,2-dihydro-3H-pyrrolo[1,2-a]pyrrole-1-carboxylic acid, is then recrystallized from ethanol/ether, m.p. 160-161 ° C.

## Claims

**Claims for the following Contracting States : AT, BE, CH, DE, FR, GB, GR, IT, LI, LU, NL, SE**

**1.** A process for producing a diester of formula XII,

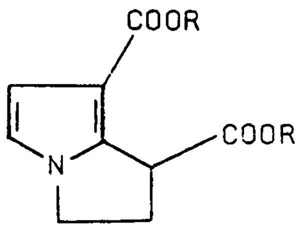

(XII)

in which
each R is independently $C_1$-$C_6$ alkyl, which comprises
(a) cyclizing a compound of formula XVI,

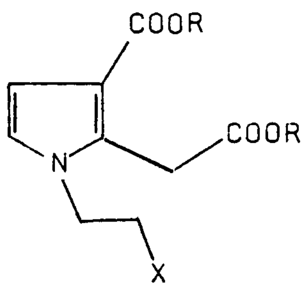

(XVI)

in which
R is as defined above; and
X is halogen,
with a hindered amine in an aprotic polar solvent; or
(b) reacting a compound of formula XVII

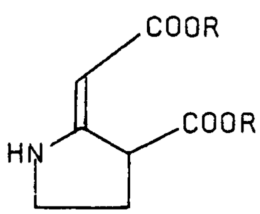

(XVII)

in which
R is as defined above,
with a 2-haloacetaldehyde, $XCH_2CHO$,
in which
X is halogen,
in aqueous solution.

**2.** The process of Claim 1 wherein each R is $CH_3$ and X is Br or Cl.

**3.** The process of Claim 2 wherein X is Br and the 2-bromoacetaldehyde is prepared by acid hydrolysis of its di($C_1$-$C_6$ alkyl) acetal.

4. The process of any one of Claims 1 to 3 which further comprises saponification of the thus-formed diester (XII) to the corresponding diacid (XII, R = H).

5. The process of Claim 4 which further comprises
(a) esterifying a compound of formula (XII) in which both R groups are hydrogen to form a compound of formula (XVIII)

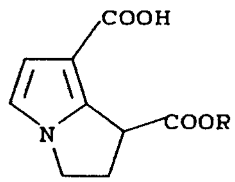

(XVIII)

in which the R group in position 1 is alkyl;
(b) decarboxylating a compound of formula (XVIII) to form a compound of formula (XIX)

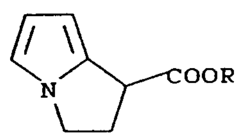

(XIX)

and
(c) aroylation of a compound of formula (XIX) with an amide or morpholide to form a compound of formula (I) or (IA)

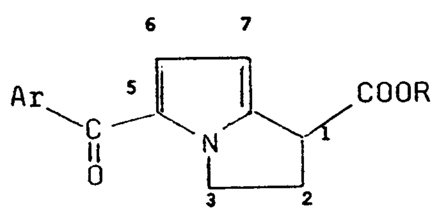

(I) or (IA)

wherein Ar is substituted or unsubstituted phenyl, 2- or 3-furyl, 2- or 3-thienyl, or 2- or 3-pyrryl and which, if substituted, can have one or more $C_1$-$C_6$ alkyl, $C_1$-$C_6$ alkoxy, or halo groups in any available position on the ring, and R is hydrogen (formula I) or alkyl of 1 to 12 carbon atoms (formula IA).

6. The process of Claim 5 which further comprises one or more of the steps:
(a) converting a compound of formula (I) to a pharmaceutically acceptable salt of a compound of formula (I); or
(b) converting a salt of a compound of formula (I) to the corresponding free compound of formula (I); or
(c) esterifying a compound of formula (I); or
(d) converting an ester of formula (IA) to the corresponding free compound of formula (I).

7. The process of Claims 5 and 6 wherein the compound formed is 5-benzoyl-1, 2-dihydro-3H-pyrrolo(1,2-a)pyrrolo-1-carboxylic acid or a pharmaceutically acceptable salt or ester thereof; or is 5-p-anisoyl-1,2-dihydro-3H-pyrrolo(1,2-a)- pyrrole-1-carboxylic acid or a pharmaceutically acceptable salt or ester thereof.

**8.** A process for producing a compound of formula XVI,

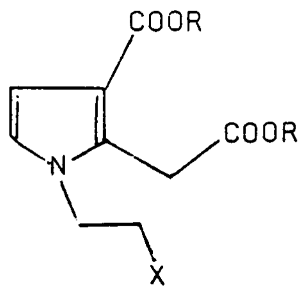

(XVI)

in which
each R is independently $C_1$-$C_6$ alkyl; and
X is halogen,
which comprises
treating a compound of formula XIII,

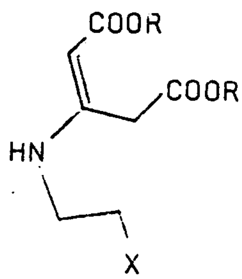

(XIII)

in which
R and X are as defined above,
with a 2-haloacetaldehyde, $XCH_2CHO$,
in which
X is halogen,
in aqueous solution.

**9.** The process of Claim 8 wherein each R is $CH_3$ and X is Br or Cl.

**10.** The process of Claim 9 wherein X is Br and the 2-bromoacetaldehyde is prepared by acid hydrolysis of its di($C_1$-$C_6$ alkyl) acetal.

**11.** A process for producing a compound of formula XVII

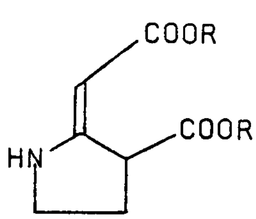

(XVII)

in which
each R is independently $C_1$-$C_6$ alkyl,
which comprises
(a) reacting compound of formula XIII

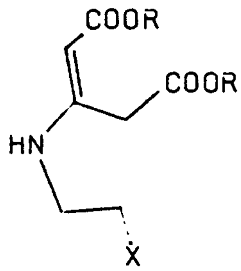

(XIII)

in which

R is as defined above; and

X is halogen,

with a strong base in an aprotic polar solvent; or

(b) reacting a compound of formula (XV)

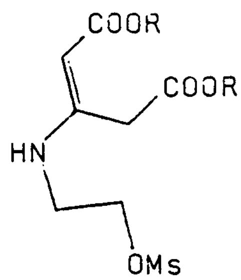

(XV)

in which Ms is mesyl,

with a phase transfer catalyst and a strong base in an aprotic solvent.

**12.** The process of Claim 11 wherein each R is selected from $CH_3$, $C_2H_5$, or n-$C_3H_7$.

**13.** A process for producing a compound of formula XIII,

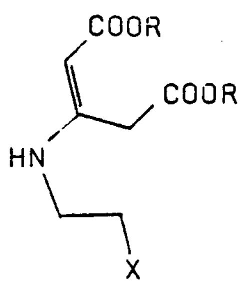

(XIII)

in which

each R is independently $C_1$-$C_6$ alkyl; and

X is halogen,

which comprises

(a) treating a di($C_1$-$C_6$ alkyl) 1,3-acetone-dicarboxylate,

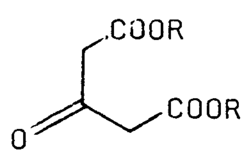

in which R is as defined above,

with a 2-haloethylamine hydrohalide salt,

$XCH_2CH_2NH_2.HX$, in which X is halogen,

in aqueous solution; or
(b) treating a di($C_1$-$C_6$ alkyl) 1,3-acetone-dicarboxylate;

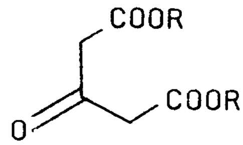

in which R is as defined above,
with $HOCH_2CH_2NH_2$ in an aprotic solvent to produce a hydroxyenamine (XIV);

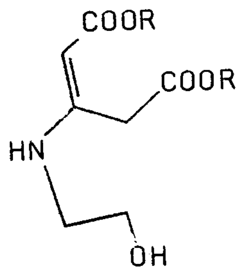

(XIV)

treating the hydroxyenamine (XIV) with mesyl chloride and a base in an aprotic solvent to produce a mesylenamine (XV);

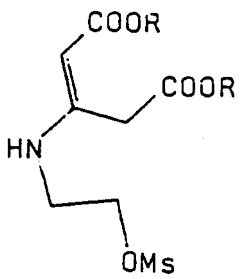

(XV)

in which Ms is mesyl,
and treating the mesylenamine (XV) with an alkali metal halide in an aprotic solvent to produce the compound of formula XIII.

**14.** The process of Claim 13 wherein each R is $CH_3$, and X is Cl or Br.

**15.** A compound of formula XIII,

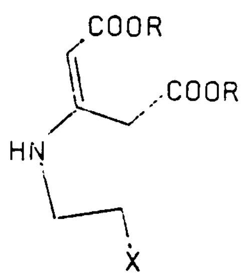

(XIII)

in which
each R is independently $C_1$-$C_6$ alkyl; and
X halogen.

**16.** The compound of Claim 15 wherein each R is selected from $CH_3$, $C_2H_5$, and n-$C_3H_7$.

**17.** The compound of Claim 15 wherein X is Cl or Br.

**18.** The compound of Claim 15 wherein each R is $CH_3$ and X is Cl.

**19.** A compound of formula XV,

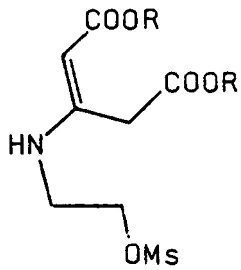

(XV)

in which
Ms is mesyl; and
each R is independently $C_1$-$C_6$ alkyl.

**20.** The compound of Claim 19 wherein each R is selected from $CH_3$, $C_2H_5$, and n-$C_3H_7$.

**21.** A compound of formula XVII

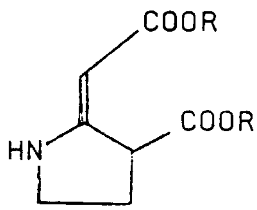

(XVII)

in which
each R is independently $C_1$-$C_6$ alkyl.

**22.** The compound of Claim 21 wherein each R is $CH_3$, $C_2H_5$, or n-$C_3H_7$.

**Claims for the following Contracting State : ES**

**1.** A process for producing a diester of formula XII,

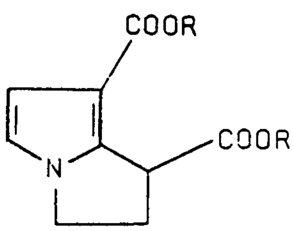

(XII)

in which
each R is independently $C_1$-$C_6$ alkyl,
which comprises
(a) cyclizing a compound of formula XVI,

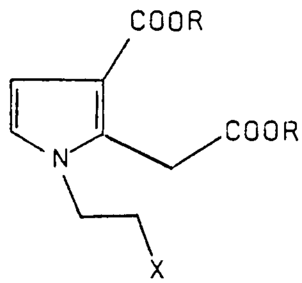

(XVI)

in which

R is as defined above; and

X is halogen,

with a hindered amine in an aprotic polar solvent; or

(b) reacting a compound of formula XVII

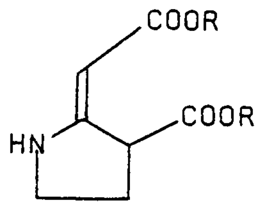

(XVII)

in which

R is as defined above,

with a 2-haloacetaldehyde, $XCH_2CHO$,

in which

X is halogen,

in aqueous solution.

**2.** The process of Claim 1 wherein each R is $CH_3$ and X is Br or Cl.

**3.** The process of Claim 2 wherein X is Br and the 2-bromoacetaldehyde is prepared by acid hydrolysis of its di($C_1$-$C_6$ alkyl) acetal.

**4.** The process of any one of Claims 1 to 3 which further comprises saponification of the thus-formed diester (XII) to the corresponding diacid (XII, R = H).

**5.** The process of Claim 4 which further comprises

(a) esterifying a compound of formula (XII) in which both R groups are hydrogen to form a compound of formula (XVIII)

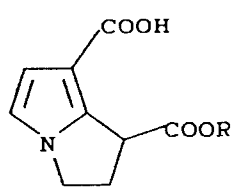

(XVIII)

in which the R group in position 1 is alkyl;

(b) decarboxylating a compound of formula (XVIII) to form a compound of formula (XIX)

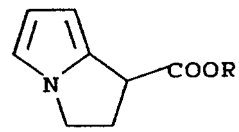

(XIX)

and

(c) aroylation of a compound of formula (XIX) with an amide or morpholide to form a compound of formula (I) or (IA)

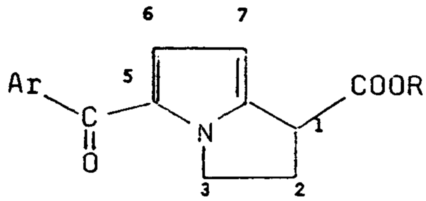

(I or (IA)

wherein Ar is substituted or unsubstituted phenyl, 2- or 3-furyl, 2- or 3-thienyl, or 2- or 3-pyrryl and which, if substituted, can have one or more $C_1$-$C_6$ alkyl, $C_1$-$C_6$ alkoxy, or halo groups in any available position on the ring, and R is hydrogen (formula I) or alkyl of 1 to 12 carbon atoms (formula IA).

**6.** The process of Claim 5 which further comprises one or more of the steps:

(a) converting a compound of formula (I) to a pharmaceutically acceptable salt of a compound of formula (I); or

(b) converting a salt of a compound of formula (I) to the corresponding free compound of formula (I); or

(c) esterifying a compound of formula (I); or

(d) converting an ester of formula (IA) to the corresponding free compound of formula (I).

**7.** The process of Claims 5 and 6 wherein the compound formed is 5-benzoyl-1,2-dihydro-3H-pyrrolo(1,2-a)- pyrrole-1-carboxylic acid or a pharmaceutically acceptable salt or a)- pyrrole-1-carboxylic acid or a pharmaceutically acceptable salt or ester thereof.

**8.** A process for producing a compound of formula XVI,

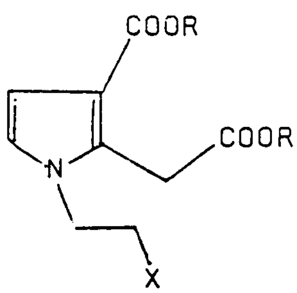

(XVI)

in which

each R is independently $C_1$-$C_6$ alkyl; and

X is halogen,

which comprises

(a) treating a compound of formula XIII,

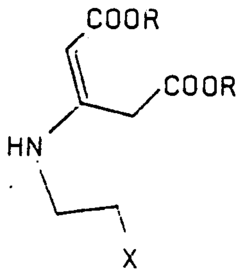

(XIII)

in which

R and X are as defined above,

with a 2-haloacetaldehyde, $XCH_2CHO$,

in which

X is halogen,

in aqueous solution.

**9.** The process of Claim 8 wherein each R is $CH_3$ and X is Br or Cl.

**10.** The process of Claim 9 wherein X is Br and the 2-bromoacetaldehyde is prepared by acid hydrolysis of its di($C_1$-$C_6$ alkyl) acetal.

**11.** A process for producing a compound of formula XVII

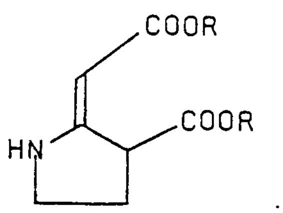

(XVII)

in which

each R is independently $C_1$-$C_6$ alkyl,

which comprises

(a) reacting compound of formula XIII

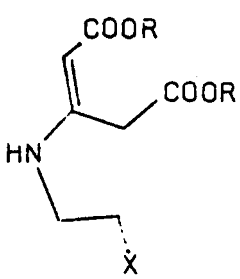

(XIII)

in which

R is as defined above; and

X is halogen,

with a strong base in an aprotic polar solvent; or

(b) reacting compound of formula (XV)

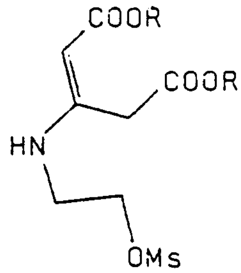

(XV)

in which Ms is mesyl,
with a phase transfer catalyst and a strong base in an aprotic solvent.

**12.** The process of Claim 11 wherein each R is selected from $CH_3$, $C_2H_5$, or n-$C_3H_7$.

**13.** A process for producing a compound of formula XIII,

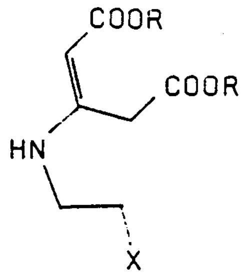

(XIII)

in which
each R is independently $C_1$-$C_6$ alkyl; and
X is halogen,
which comprises
(a) treating a di($C_1$-$C_6$ alkyl) 1,3-acetone-dicarboxylate,

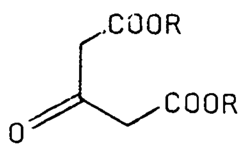

in which R is as defined above,
with a 2-haloethylamine hydrohalide salt,
$XCH_2CH_2NH_2$.HX, in which X is halogen,
in aqueous solution; or
(b) treating a di($C_1$-$C_6$ alkyl) 1,3-acetone-dicarboxylate:

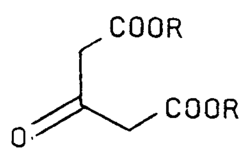

in which R is as defined above,
with $HOCH_2CH_2NH_2$ in an aprotic solvent to produce a hydroxyenamine (XIV);

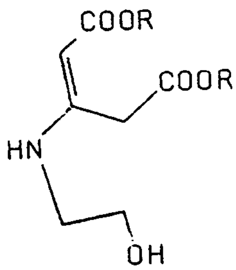

(XIV)

treating the hydroxyenamine (XIV) with mesyl chloride and a base in an aprotic solvent to produce a mesylenamine (XV);

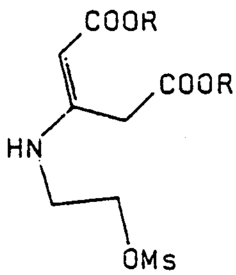

(XV)

in which Ms is mesyl,
and treating the mesylenamine (XV) with an alkali metal halide in an aprotic solvent to produce the compound of formula XIII.

**14.** The process of Claim 13 wherein each R is selected from $CH_3$, $C_2H_5$, and n-$C_3H_7$.

**15.** The process of Claim 13 or 14 wherein X is Cl or Br.

**16.** The process of Claim 15 wherein each R is $CH_3$ and X is Cl.

**17.** A process for producing a compound of formula XV

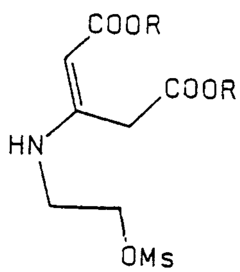

(XV)

in which
Ms is mesyl; and
each R is independently $C_1$-$C_6$ alkyl,
which comprises treating a di($C_1$-$C_6$ alkyl) 1, 3-acetone-dicarboxylate,

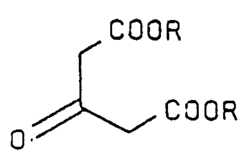

in which R is as defined above,
with $HOCH_2CH_2NH_2$ in an aprotic solvent to produce a hydroxy-enamine (XIV);

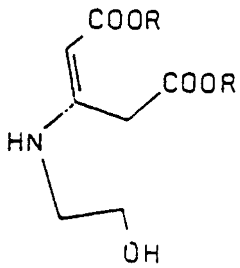

(XIV)

and

treating the hydroxyenamine (XIV) with mesyl chloride and a base in an aprotic solvent.

**18.** The process of Claim 17 wherein each R is selected from $CH_3$, $C_2H_5$. and n-$C_3H_7$.

**Revendications**

**Revendications pour les Etats contractants suivants : AT, BE, CH, DE, FR, GB, GR, IT, LI, LU, NL, SE**

**1.** Procédé de production d'un diester de formule XII,

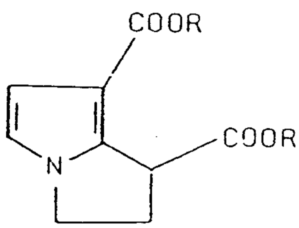

(XII)

dans laquelle

chaque groupe R représente, indépendamment, un groupe alkyle en $C_1$ à $C_6$,

qui consiste

(a) à cycliser un composé de formule XVI,

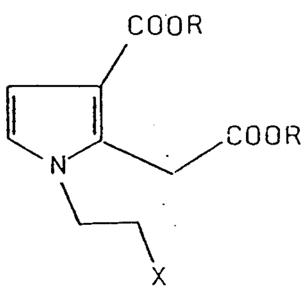

(XVI)

dans laquelle

R répond à la définition précitée ; et

X représente un halogène,

avec une amine à encombrement stérique dans un solvant polaire aprotique ; ou

(b) à faire réagir un composé de formule XVII

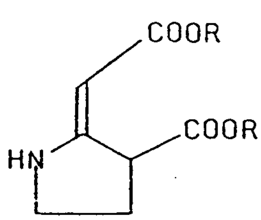

(XVII)

dans laquelle
R répond à la définition précitée,
avec un 2-halogénacétaldéhyde de formule $XCH_2CHO$,
dans laquelle
X représente un halogène,
en solution aqueuse.

2. Procédé suivant la revendication 1, dans lequel chaque groupe R représente un groupe $CH_3$ et X représente Br ou Cl.

3. Procédé suivant la revendication 2, dans lequel X représente Br et le 2-bromacétaldéhyde est préparé par hydrolyse acide de son di-(alkyle en $C_1$ à $C_6$)-acétal.

4. Procédé suivant l'une quelconque des revendications 1 à 3, qui consiste en outre à saponifier le diester (XII) ainsi formé en le diacide correspondant (XII, R = H).

5. Procédé suivant la revendication 4, qui consiste en outre
(a) à estérifier un composé de formule (XII), dans laquelle les deux groupes R représentent l'hydrogène, pour former un composé de formule (XVIII)

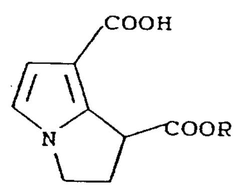

(XVIII)

dans laquelle le groupe R en position 1 représente un groupe alkle ;
(b) à décarboxyler un composé de formule (XVIII) pour former un composé de formule (XIX)

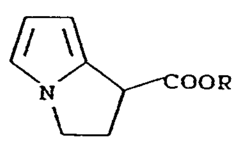

(XIX)

(c) à aroyler un composé de formule (XIX) avec un amide ou morpholide pour former un composé de formule (I) ou (IA)

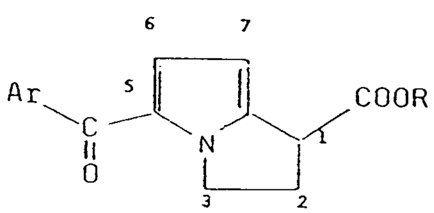

(I) ou (IA)

dans laquelle Ar représente un groupe phényle, 2- ou 3-furyle, 2- ou 3-thiényle ou 2- ou 3-pyrryle substitué ou non substitué et qui, s'il est substitué, peut porter un ou plusieurs groupes alkyle en $C_1$ à $C_6$, alkoxy en $C_1$ à $C_6$ ou halogéno dans n'importe quelle position disponible sur le noyau, et R représente l'hydrogène (formule I) ou un groupe alkyle ayant 1 à 12 atomes de carbone (formule IA).

**6.** Procédé suivant la revendication 5, qui comprend en outre une ou plusieurs des étapes consistant :
(a) à transformer un composé de formule (I) en un sel pharmaceutiquement acceptable d'un composé de formule (I) ; ou
(b) à transformer un sel d'un composé de formule (I) en le composé libre correspondant de formule (I) ; ou
(c) à estérifier un composé de formule (I) ; ou bien
(d) à transformer un ester de formule (IA) en le composé libre correspondant de formule (I).

**7.** Procédé suivant les revendications 5 et 6, dans lequel le composé formé est l'acide 5-benzoyl-1,2-dihydro-3H-pyrrolo(1,2-a)pyrrolo-1-carboxylique ou un de ses sels ou esters pharmaceutiquement acceptables ou bien l'acide 5-p-anisoyl-1,2-dihydro-3H-pyrrolo(1,2-a)-pyrrole-1-carboxylique ou un de ses sels ou esters pharmaceutiquement acceptables.

**8.** Procédé de production d'un composé de formule XVI,

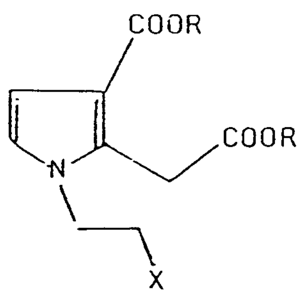

(XVI)

dans laquelle
chaque groupe R représente, indépendamment, un groupe alkyle en $C_1$ à $C_6$ ; et
X représente un halogène,
qui consiste
à traiter un composé de formule XIII,

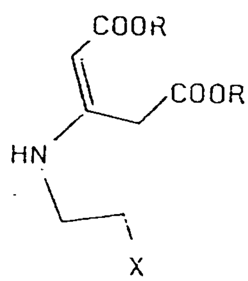

(XIII)

dans laquelle
R et X répondent aux définitions précitées,
avec un 2-halogénacétaldéhyde de formule $XCH_2CHO$,
dans laquelle
X représente un halogène,
en solution aqueuse.

**9.** Procédé suivant la revendication 8, dans lequel chaque groupe R représente un groupe $CH_3$ et X représente Br ou Cl.

**10.** Procédé suivant la revendication 9, dans lequel X représente Br et le 2-bromacétaldéhyde est préparé par hydrolyse acide de son di-(alkyle en $C_1$ à $C_6$)-acétal.

**11.** Procédé de production d'un composé de formule XVII

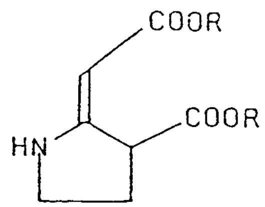

(XVII)

dans laquelle

chaque groupe R représente, indépendamment, un groupe alkyle en $C_1$ à $C_6$,

qui consiste

(a) à faire réagir un composé de formule XIII

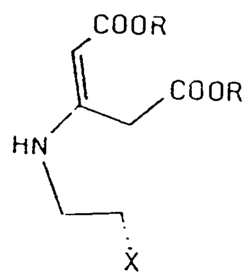

(XIII)

dans laquelle

R répond à la définition précitée ; et

X représente un halogène,

avec une base forte dans un solvant polaire aprotique ; ou bien

(b) à faire réagir un composé de formule (XV)

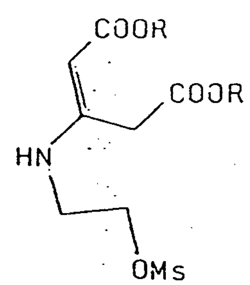

(XV)

dans laquelle Ms représente un groupe mésyle,

avec un catalyseur de transfert de phase et une base forte dans un solvant aprotique.

**12.** Procédé suivant la revendication 11, dans lequel chaque groupe R est choisi entre les groupes $CH_3$, $C_2H_5$ et n-$C_3H_7$.

**13.** Procédé de production d'un composé de formule XIII,

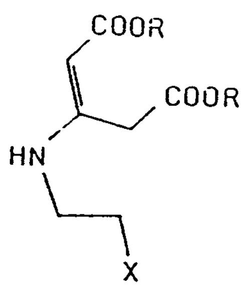

(XIII)

dans laquelle

chaque groupe R représente, indépendamment, un groupe alkyle en $C_1$ à $C_6$ ; et
X représente un halogène,
qui consiste
(a) à traiter un 1,3-acétone-dicarboxylate de di-(alkyle en $C_1$ à $C_6$)

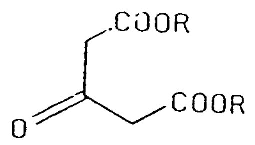

dans lequel R répond à la définition précitée,
avec un halogénhydrate de 2-halogénéthylamine de formule $XCH_2CH_2NH_2.HX$, dans laquelle X représente un halogène,
en solution aqueuse ; ou bien
(b) à traiter un 1,3-acétone-dicarboxylate de di-(alkyle en $C_1$ à $C_6$)

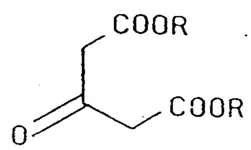

dans lequel R répond à la définition précitée,
avec un composé de formule $HOCH_2CH_2NH_2$ dans un solvant aprotique pour produire une hydroxyénamine (XIV) ;

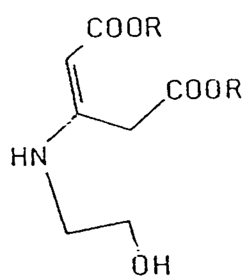

(XIV)

à traiter l'hydroxyénamine (XIV) avec le chlorure de mésyle et une base dans un solvant aprotique pour produire une mésylénamine (XV) ;

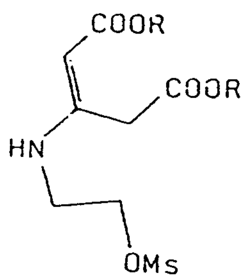

(XV)

dans laquelle Ms représente un groupe mésyle,
et à traiter la mésylénamine (XV) avec un halogénure de métal alcalin dans un solvant aprotique pour produire le composé de formule XIII.

**14.** Procédé suivant la revendication 13, dans lequel chaque groupe R représente un groupe $CH_3$ et X représente Cl ou Br.

**15.** Composé de formule XIII,

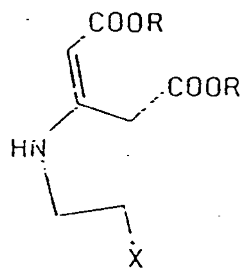

(XIII)

dans laquelle
chaque groupe R représente, indépendamment, un groupe alkyle en $C_1$ à $C_6$ ; et
X représente un halogène.

**16.** Composé suivant la revendication 15, dans lequel chaque groupe R est choisi entre les groupes $CH_3$, $C_2H_5$ et n-$C_3H_7$.

**17.** Composé suivant la revendication 15, dans lequel X représente Cl ou Br.

**18.** Composé suivant la revendication 15, dans lequel chaque groupe R représente un groupe $CH_3$ et X représente Cl.

**19.** Composé de formule XV,

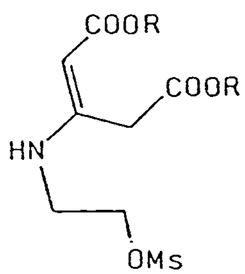

(XV)

dans laquelle
Ms représente un groupe mésyle ; et
chaque groupe R représente, indépendamment, un groupe alkyle en $C_1$ à $C_6$.

**20.** Composé suivant la revendication 19, dans lequel chaque groupe R est choisi entre les groupes $CH_3$, $C_2H_5$ et n-$C_3H_7$.

**21.** Composé de formule XVII

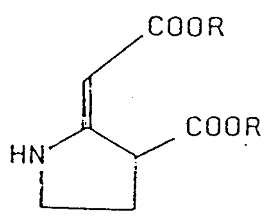

(XVII)

dans laquelle
chaque groupe R représente, indépendamment, un groupe alkyle en $C_1$ à $C_6$.

**22.** Composé suivant la revendication 21, dans lequel chaque groupe R représente un groupe $CH_3$, $C_2H_5$ ou n-$C_3H_7$.

**Revendications pour l'Etat contractant suivant : ES**

**1.** Procédé de production d'un diester de formule XII,

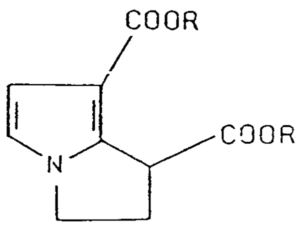

(XII)

dans laquelle
chaque groupe R représente, indépendamment, un groupe alkyle en $C_1$ à $C_6$,
qui consiste
(a) à cycliser un composé de formule XVI,

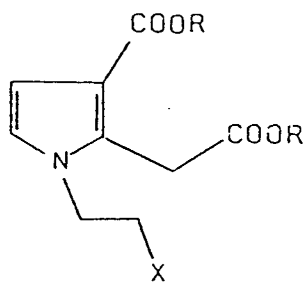

(XVI)

dans laquelle
R répond à la définition précitée ; et
X représente un halogène,
avec une amine à encombrement stérique dans un solvant polaire aprotique ; ou bien
(b) à faire réagir un composé de formule XVII

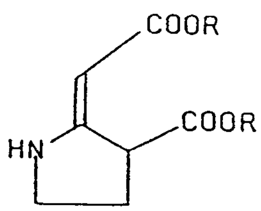

(XVII)

dans laquelle
R répond à la définition précitée,
avec un 2-halogénacétaldéhyde de formule $XCH_2CHO$,
dans laquelle
X représente un halogène,
en solution aqueuse.

**2.** Procédé suivant la revendication 1, dans lequel chaque groupe R représente un groupe $CH_3$ et X représente Br ou Cl.

**3.** Procédé suivant la revendication 2, dans lequel X représente Br et le 2-bromacétaldéhyde est préparé par hydrolyse acide de son di-(alkyle en $C_1$ à $C_6$)-acétal.

**4.** Procédé suivant l'une quelconque des revendications 1 à 3, qui consiste en outre à saponifier le diester (XII) ainsi formé en le diacide correspondant (XII, R = H).

**5.** Procédé suivant la revendication 4, qui consiste en outre
(a) à estérifier un composé de formule (XII), dans laquelle les deux groupes R représentent

l'hydrogène, pour former un composé de formule (XVIII)

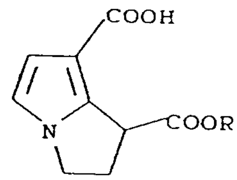

(XVIII)

dans laquelle le groupe R en position 1 représente un groupe alkyle ;
(b) à décarboxyler un composé de formule (XVIII) pour former un composé de formule (XIX)

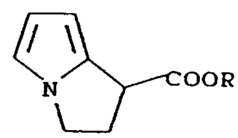

(XIX)

et
(c) à aroyler un composé de formule (XIX) avec un amide ou morpholide pour former un composé de formule (I) ou (IA)

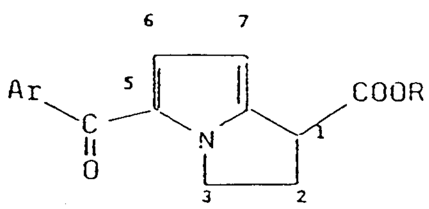

(I) ou (IA)

dans laquelle Ar représente un groupe phényle, 2- ou 3-furyle, 2- ou 3-thiényle ou 2- ou 3-pyrryle substitué ou non substitué et qui, s'il est substitué, peut porter un ou plusieurs groupes alkyle en $C_1$ à $C_6$, alkoxy en $C_1$ à $C_6$ ou halogéno dans n'importe quelle position disponible sur le noyau et R représente l'hydrogène (formule I) ou un groupe alkyle ayant 1 à 12 atomes de carbone (formule IA).

**6.** Procédé suivant la revendication 5, qui comprend en outre une ou plusieurs des étapes consistant :
(a) à transformer un composé de formule (I) en un sel pharmaceutiquement acceptable d'un composé de formule (I) ; ou
(b) à transformer un sel d'un composé de formule (I) en le composé libre correspondant de formule (I) ; ou
(c) à estérifier un composé de formule (I) ; ou bien
(d) à transformer un ester de formule (IA) en le composé libre correspondant de formule (I).

**7.** Procédé suivant les revendications 5 et 6, dans lequel le composé formé est l'acide 5-benzoyl-1,2-dihydro-3H-pyrrolo(1,2-a)pyrrole-1-carboxylique ou un de ses sels ou esters pharmaceutiquement acceptables ; ou bien l'acide 5-p-anisoyl-1,2-dihydro-3H-pyrrolo(1,2-a)-pyrrole-1-carboxylique ou un de ses sels ou esters pharmaceutiquement acceptables.

**8.** Procédé de production d'un composé de formule XVI,

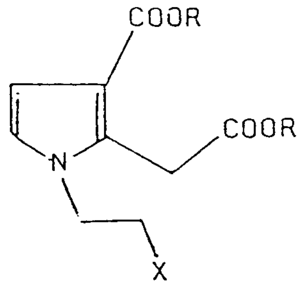

(XVI)

dans laquelle

chaque groupe R représente, indépendamment, un groupe alkyle en $C_1$ à $C_6$ ; et

X représente un halogène,

qui consiste

(a) à traiter un composé de formule XIII,

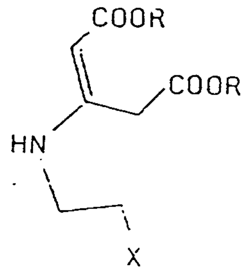

(XIII)

dans laquelle

R et X répondent aux définitions précitées,

avec un 2-halogénacétaldéhyde de formule $XCH_2CHO$,

dans laquelle

X représente un halogène,

en solution aqueuse.

**9.** Procédé suivant la revendication 8, dans lequel chaque groupe R représente un groupe $CH_3$ et X représente Br ou Cl.

**10.** Procédé suivant la revendication 9, dans lequel X représente Br et le 2-bromacétaldéhyde est préparé par hydrolyse acide de son di-(alkyle en $C_1$ à $C_6$)-acétal.

**11.** Procédé de production d'un composé de formule XVII

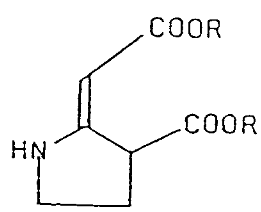

(XVII)

dans laquelle

chaque groupe R représente, indépendamment, un groupe alkyle en $C_1$ à $C_6$,

qui consiste

(a) à faire réagir un composé de formule XIII

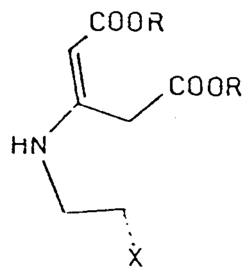

(XIII)

dans laquelle
R répond à la définition précitée ; et
X représente un halogène,
avec une base forte dans un solvant polaire aprotique ; ou bien
(b) à faire réagir un composé de formule (XV)

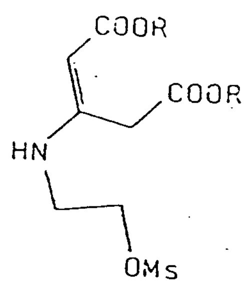

(XV)

dans laquelle Ms représente un groupe mésyle,
avec un catalyseur de transfert de phase et une base forte dans un solvant aprotique.

**12.** Procédé suivant la revendication 11, dans lequel chaque groupe R est choisi entre les groupes $CH_3$, $C_2H_5$ et n-$C_3H_7$.

**13.** Procédé de production d'un composé de formule XIII,

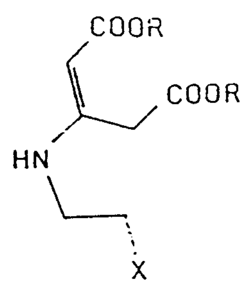

(XIII)

dans laquelle
chaque groupe R représente, indépendamment, un groupe alkyle en $C_1$ à $C_6$ ; et
X représente un halogène,
qui consiste
(a) à traiter un 1,3-acétone-dicarboxylate de di-(alkyle en $C_1$ à $C_6$) de formule

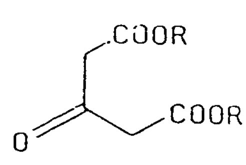

dans lequel R répond à la définition précitée,
avec un halogénhydrate de 2-halogénéthylamine de formule $XCH_2CH_2NH_2.HX$, dans laquelle X

représente un halogène,
en solution aqueuse ; ou bien
(b) à traiter un 1,3-acétone-dicarboxylate de di-(alkyle en $C_1$ à $C_6$) de formule

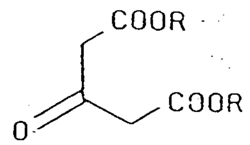

dans lequel R répond à la définition précitée,
avec un composé de formule $HOCH_2CH_2NH_2$ dans un solvant aprotique pour produire une hydroxyénamine de formule (XIV) ;

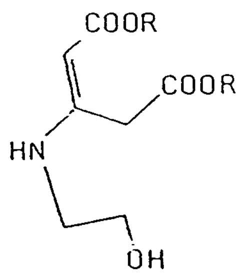

(XIV)

à traiter l'hydroxyénamine (XIV) avec le chlorure de mésyle et une base dans un solvant aprotique pour produire une mésylénamine de formule (XV) ;

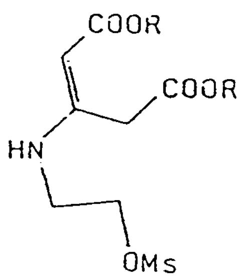

(XV)

dans laquelle Ms représente un groupe mésyle,
et à traiter la mésylénamine (XV) avec un halogénure de métal alcalin dans un solvant aprotique pour produire le composé de formule XIII.

**14.** Procédé suivant la revendication 13, dans lequel chaque groupe R est choisi entre les groupes $CH_3$, $C_2H_5$ et n-$C_3H_7$.

**15.** Procédé suivant la revendication 13 ou 14, dans lequel X représente Cl ou Br.

**16.** Procédé suivant la revendication 15, dans lequel chaque groupe R représente un groupe $CH_3$ et X représente Cl.

**17.** Procédé de production d'un composé de formule XV,

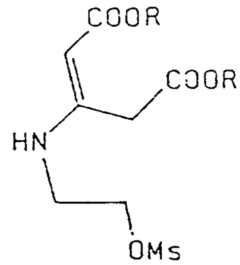

(XV)

dans laquelle

Ms représente un groupe mésyle ; et

chaque groupe R représente, indépendamment, un groupe alkyle en $C_1$ à $C_6$,

qui consiste à traiter un 1,3-acétone-dicarboxylate de di-(alkyle en $C_1$ à $C_6$) de formule

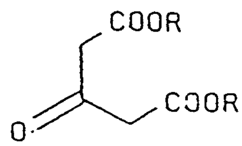

dans laquelle R répond à la définition précitée,

avec un composé de formule $HOCH_2CH_2NH_2$ dans un solvant aprotique pour produire une hydroxyénamine de formule (XIV)

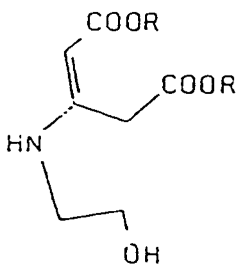

(XIV)

et

à traiter l'hydroxyénamine (XIV) avec le chlorure de mésyle et une base dans un solvant aprotique.

**18.** Procédé suivant la revendication 17, dans lequel chaque R est choisi entre les groupes $CH_3$, $C_2H_5$ et n-$C_3H_7$.

**Patentansprüche**

**Patentansprüche für folgende Vertragsstaaten : AT, BE, CH, DE, FR, GB, GR, IT, LI, LU, NL, SE**

**1.** Verfahren zur Herstellung eines Diesters der Formel XII,

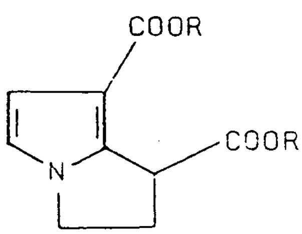

(XII)

worin

jedes R unabhängig $C_1$-$C_6$-Alkyl ist,

umfassend

(a) das Cyclisieren einer Verbindung der Formel XVI

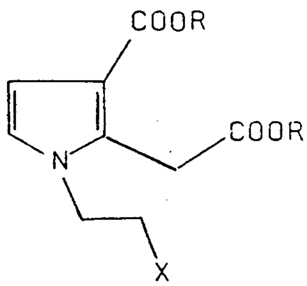

(XVI)

worin
R wie oben definiert ist; und
X Halogen ist,
mit einem gehinderten Amin in einem aprotischen polaren Lösungsmittel; oder
(b) das Umsetzen einer Verbindung der Formel XVII

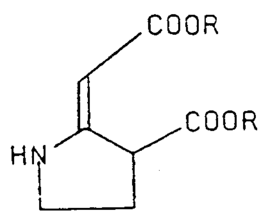

(XVII)

worin
R wie oben definiert ist,
mit einem 2-Halogenacetaldehyd, $XCH_2CHO$,
worin
X Halogen ist,
in wäßriger Lösung.

2. Verfahren nach Anspruch 1, worin jedes R $CH_3$ ist und X Br oder Cl ist.

3. Verfahren nach Anspruch 2, worin X Br ist und der 2-Bromacetaldehyd durch saure Hydrolyse seines Di($C_1$-$C_6$-alkyl) acetals hergestellt wird.

4. Verfahren nach irgendeinem der Ansprüche 1 bis 3, das weiter die Verseifung des dadurch gebildeten Diesters (XII) zu der entsprechenden Disäure (XII, R = H) umfaßt.

5. Verfahren nach Anspruch 4, weiter umfassend
(a) das Verestern einer Verbindung der Formel (XII), worin beide Gruppen R Wasserstoff sind, um eine Verbindung der Formel (XVIII) zu bilden

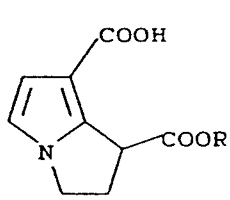

(XVIII)

worin die Gruppe R an Position 1 Alkyl ist;
(b) das Decarboxylieren einer Verbindung der Formel (XVIII), um eine Verbindung der Formel (XIX)

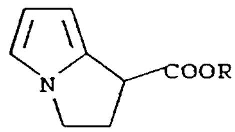

(XIX)

zu bilden, und

(c) die Aroylierung einer Verbindung der Formel (XIX) mit einem Amid oder Morpholid, um eine Verbindung der Formel (I) oder (IA)

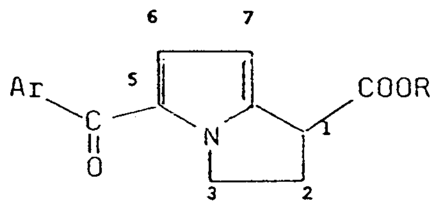

(I) oder (IA)

zu bilden, worin Ar substituiertes oder unsubstituiertes Phenyl, 2- oder 3-Furyl, 2- oder 3-Thienyl oder 2- oder 3-Pyrryl ist und welches, falls es substituiert ist, eine oder mehrere $C_1$-$C_6$-Alkyl-, $C_1$-$C_6$-Alkoxy- oder Halogengruppen an irgendeiner zur Verfügung stehenden Position des Rings aufweisen kann und R Wasserstoff (Formel I) oder Alkyl mit 1 bis 12 Kohlenstoffatomen (Formel IA) ist.

**6.** Verfahren nach Anspruch 5, weiter umfassend eine oder mehrere der Stufen:

(a) das Überführen einer Verbindung der Formel (I) in ein pharmazeutisch annehmbares Salz einer Verbindung der Formel (I); oder

(b) das Überführen eines Salzes einer Verbindung der Formel (I) in die entsprechende freie Verbindung der Formel (I); oder

(c) das Verestern einer Verbindung der Formel (I); oder

(d) das Überführen eines Esters der Formel (IA) in die entsprechende freie Verbindung der Formel (I).

**7.** Verfahren nach den Ansprüchen 5 und 6, worin die gebildete Verbindung 5-Benzoyl-1,2-dihydro-3H-pyrrolo(1,2-a)pyrrolo-1-carbonsäure oder ein pharmazeutisch annehmbares Salz oder ein pharmazeutisch annehmbarer Ester davon ist; oder 5-p-Anisoyl-1,2-dihydro-3H-pyrrolo(1,2-a)-pyrrol-1-carbonsäure oder ein pharmazeutisch annehmbares Salz oder ein pharmazeutisch annehmbarer Ester davon ist.

**8.** Verfahren zur Herstellung einer Verbindung der Formel XVI,

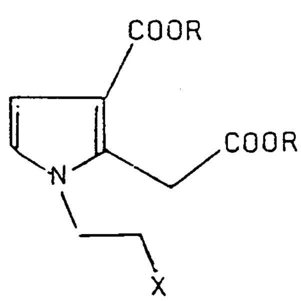

(XVI)

worin

jedes R unabhängig $C_1$-$C_6$-Alkyl ist; und

X Halogen ist,

umfassend

das Behandeln einer Verbindung der Formel XIII

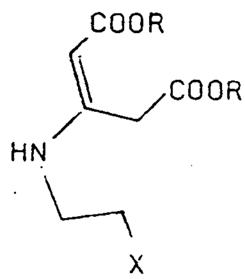

(XIII)

worin
R und X wie oben definiert sind,
mit einem 2-Halogenacetaldehyd, $XCH_2CHO$,
worin
X Halogen ist,
in wäßriger Lösung.

**9.** Verfahren nach Anspruch 8, worin jedes R $CH_3$ ist und X Br oder Cl ist.

**10.** Verfahren nach Anspruch 9, worin X Br ist und der 2-Bromacetaldehyd durch saure Hydrolyse seines Di($C_1$-$C_6$-alkyl) acetals hergestellt wird.

**11.** Verfahren zur Herstellung einer Verbindung der Formel XVII

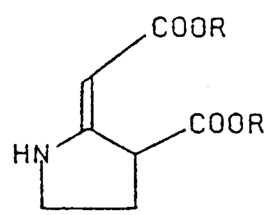

(XVII)

worin
jedes R unabhängig $C_1$-$C_6$-Alkyl ist,
umfassend
(a) das Umsetzen einer Verbindung der Formel XIII

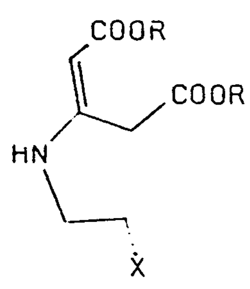

(XIII)

worin
R wie oben definiert ist; und
X Halogen ist,
mit einer starken Base in einem aprotischen polaren Lösungsmittel; oder
(b) das Umsetzen einer Verbindung der Formel (XV)

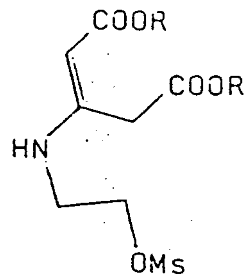

(XV)

worin Ms Mesyl ist, mit einem Phasentransfer-Katalysator und einer starken Base in einem aprotischen Lösungsmittel.

**12.** Verfahren nach Anspruch 11, worin jedes R ausgewählt ist aus $CH_3$, $C_2H_5$ oder n-$C_3H_7$.

**13.** Verfahren zur Herstellung einer Verbindung der Formel XIII,

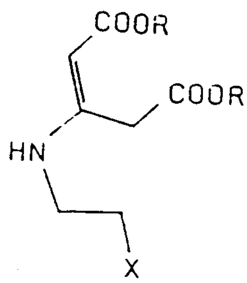

(XIII)

worin
jedes R unabhängig $C_1$-$C_6$-Alkyl ist; und
X Halogen ist,
umfassend
(a) das Behandeln eines Di($C_1$-$C_6$-alkyl)-1,3-acetondicarboxylats,

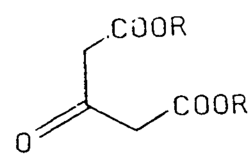

worin R wie oben definiert ist, mit einem 2-Halogenethylaminhydrohalogenidsalz, $XCH_2CH_2NH_2 \cdot HX$, worin X Halogen ist, in wäßriger Lösung; oder
(b) das Behandeln eines Di($C_1$-$C_6$-alkyl)-1,3-acetondicarboxylats;

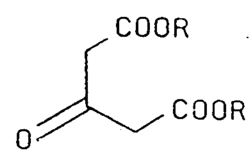

worin R wie oben definiert ist, mit $HOCH_2CH_2NH_2$ in einem aprotischen Lösungsmittel, um ein Hydroxyenamin (XIV) zu erzeugen;

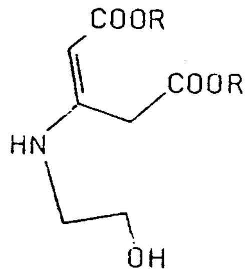

(XIV)

das Behandeln des Hydroxyenamins (XIV) mit Mesylchlorid und einer Base in einem aprotischen Lösungsmittel, um ein Mesylenamin (XV) zu erzeugen;

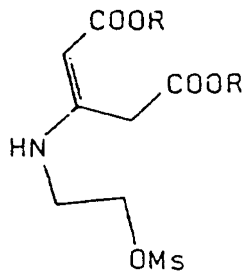

(XV)

worin Ms Mesyl ist,
und das Behandeln des Mesylenamins (XV) mit einem Alkalimetallhalogenid in einem aprotischen Lösungsmittel, um die Verbindung der Formel XIII zu erzeugen.

**14.** Verfahren nach Anspruch 13, worin jedes R $CH_3$ ist und X Cl oder Br ist.

**15.** Verbindung der Formel XIII

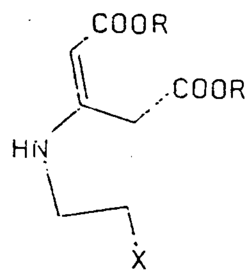

(XIII)

worin
jedes R unabhängig $C_1$-$C_6$-Alkyl ist; und
X Halogen ist.

**16.** Verbindung nach Anspruch 15, worin jedes R ausgewählt ist aus $CH_3$, $C_2H_5$ und n-$C_3H_7$.

**17.** Verbindung nach Anspruch 15, worin X Cl oder Br ist.

**18.** Verbindung nach Anspruch 15, worin jedes R $CH_3$ ist und X Cl ist.

**19.** Verbindung der Formel XV,

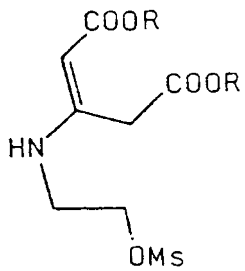

(XV)

worin
Ms Mesyl ist; und
jedes R unabhängig $C_1$-$C_6$-Alkyl ist.

**20.** Verbindung nach Anspruch 19, worin jedes R ausgewählt ist aus $CH_3$, $C_2H_5$ und n-$C_3H_7$.

**21.** Verbindung der Formel XVII

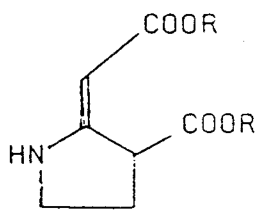

(XVII)

worin
jedes R unabhängig $C_1$-$C_6$-Alkyl ist.

**22.** Verbindung nach Anspruch 21, worin jedes R $CH_3$, $C_2H_5$ oder n-$C_3H_7$ ist.

**Patentansprüche für folgenden Vertragsstaat : ES**

**1.** Verfahren zur Herstellung eines Diesters der Formel XII,

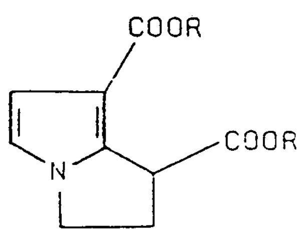

(XII)

worin
jedes R unabhängig $C_1$-$C_6$-Alkyl ist,
umfassend
(a) das Cyclisieren einer Verbindung der Formel XVI

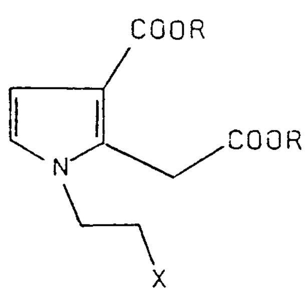

(XVI)

worin
R wie oben definiert ist; und
X Halogen ist,
mit einem gehinderten Amin in einem aprotischen polaren Lösungsmittel; oder
(b) das Umsetzen einer Verbindung der Formel XVII

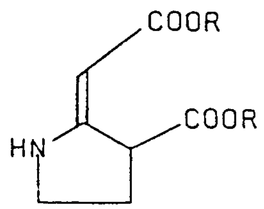

(XVII)

worin
R wie oben definiert ist,
mit einem 2-Halogenacetaldehyd, $XCH_2CHO$,
worin
X Halogen ist,
in wäßriger Lösung.

2. Verfahren nach Anspruch 1, worin jedes R $CH_3$ ist und X Br oder Cl ist.

3. Verfahren nach Anspruch 2, worin X Br ist und der 2-Bromacetaldehyd durch saure Hydrolyse seines Di($C_1$-$C_6$-alkyl) acetals hergestellt wird.

4. Verfahren nach irgendeinem der Ansprüche 1 bis 3, das weiter die Verseifung des dadurch gebildeten Diesters (XII) zu der entsprechenden Disäure (XII, R = H) umfaßt.

5. Verfahren nach Anspruch 4, weiter umfassend
(a) das Verestern einer Verbindung der Formel (XII), worin beide Gruppen R Wasserstoff sind, um eine Verbindung der Formel (XVIII) zu bilden

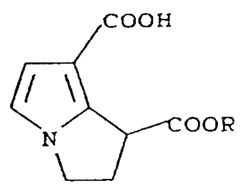

(XVIII)

worin die Gruppe R an Position 1 Alkyl ist;
(b) das Decarboxylieren einer Verbindung der Formel (XVIII), um eine Verbindung der Formel (XIX)

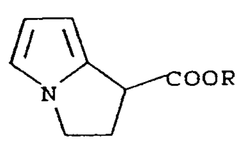

(XIX)

zu bilden, und
(c) die Aroylierung einer Verbindung der Formel (XIX) mit einem Amid oder Morpholid, um eine Verbindung der Formel (I) oder (IA)

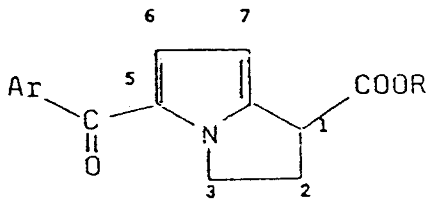

(I) oder (IA)

zu bilden, worin Ar substituiertes oder unsubstituiertes Phenyl, 2- oder 3-Furyl, 2- oder 3-Thienyl oder 2- oder 3-Pyrryl ist und welches, falls es substituiert ist, eine oder mehrere $C_1$-$C_6$-Alkyl-, $C_1$-$C_6$-Alkoxy- oder Halogengruppen an irgendeiner zur Verfügung stehenden Position des Rings aufweisen kann und R Wasserstoff (Formel I) oder Alkyl mit 1 bis 12 Kohlenstoffatomen (Formel IA) ist.

**6.** Verfahren nach Anspruch 5, weiter umfassend eine oder mehrere der Stufen:
(a) das Überführen einer Verbindung der Formel (I) in ein pharmazeutisch annehmbares Salz einer Verbindung der Formel (I); oder
(b) das Überführen eines Salzes einer Verbindung der Formel (I) in die entsprechende freie Verbindung der Formel (I); oder
(c) das Verestern einer Verbindung der Formel (I); oder
(d) das Überführen eines Esters der Formel (IA) in die entsprechende freie Verbindung der Formel (I).

**7.** Verfahren nach den Ansprüchen 5 und 6, worin die gebildete Verbindung 5-Benzoyl-1,2-dihydro-3H-pyrrolo(1,2-a)pyrrol-1-carbonsäure oder ein pharmazeutisch annehmbares Salz oder ein pharmazeutisch annehmbarer Ester davon ist; oder 5-p-Anisoyl-1,2-dihydro-3H-pyrrolo(1,2-a)-pyrrol-1-carbonsäure oder ein pharmazeutisch annehmbares Salz oder ein pharmazeutisch annehmbarer Ester davon ist.

**8.** Verfahren zur Herstellung einer Verbindung der Formel XVI,

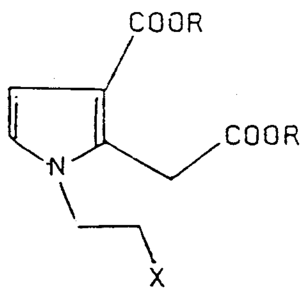

(XVI)

worin
jedes R unabhängig $C_1$-$C_6$-Alkyl ist; und
X Halogen ist,
umfassend (a) das Behandeln einer Verbindung der Formel XIII

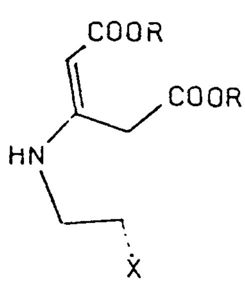

(XIII)

worin
R und X wie oben definiert sind,
mit einem 2-Halogenacetaldehyd, $XCH_2CHO$,

worin
X Halogen ist,
in wäßriger Lösung.

**9.** Verfahren nach Anspruch 8, worin jedes R $CH_3$ ist und X Br oder Cl ist.

**10.** Verfahren nach Anspruch 9, worin X Br ist und der 2-Bromacetaldehyd durch saure Hydrolyse seines Di($C_1$-$C_6$-alkyl) acetals hergestellt wird.

**11.** Verfahren zur Herstellung einer Verbindung der Formel XVII

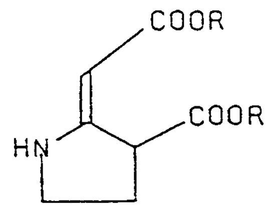

(XVII)

worin
jedes R unabhängig $C_1$-$C_6$-Alkyl ist,
umfassend
(a) das Umsetzen einer Verbindung der Formel XIII

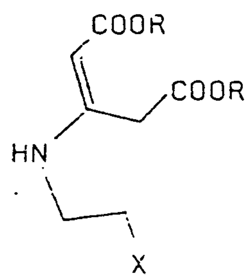

(XIII)

worin
R wie oben definiert ist; und
X Halogen ist,
mit einer starken Base in einem aprotischen polaren Lösungsmittel; oder
(b) das Umsetzen einer Verbindung der Formel (XV)

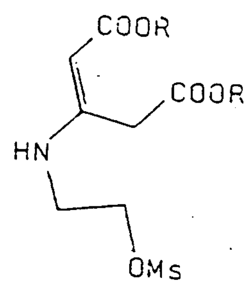

(XV)

worin Ms Mesyl ist,
mit einem Phasentransfer-Katalysator und einer starken Base in einem aprotischen Lösungsmittel.

**12.** Verfahren nach Anspruch 11, worin jedes R ausgewählt ist aus $CH_3$, $C_2H_5$ oder n-$C_3H_7$.

**13.** Verfahren zur Herstellung einer Verbindung der Formel XIII,

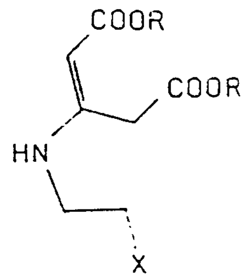

(XIII)

worin
jedes R unabhängig $C_1$-$C_6$-Alkyl ist; und
X Halogen ist,
umfassend
(a) das Behandeln eines Di($C_1$-$C_6$-alkyl)-1,3-acetondicarboxylats,

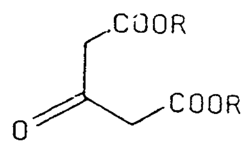

worin R wie oben definiert ist,
mit einem 2-Halogenethylaminhydrohalogenidsalz, $XCH_2CH_2NH_2 \cdot HX$, worin X Halogen ist, in wäßriger Lösung; oder
(b) das Behandeln eines Di($C_1$-$C_6$-alkyl)-1,3-acetondicarboxylats;

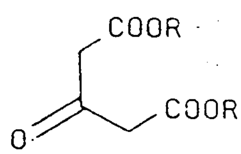

worin R wie oben definiert ist,
mit $HOCH_2CH_2NH_2$ in einem aprotischen Lösungsmittel, um ein Hydroxyenamin (XIV) zu erzeugen;

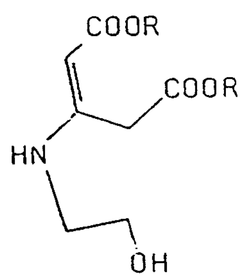

(XIV)

das Behandeln des Hydroxyenamins (XIV) mit Mesylchlorid und einer Base in einem aprotischen Lösungsmittel, um ein Mesylenamin (XV) zu erzeugen;

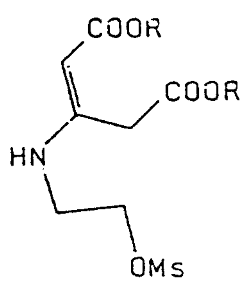

(XV)

worin Ms Mesyl ist,
und das Behandeln des Mesylenamins (XV) mit einem Alkalimetallhalogenid in einem aprotischen Lösungsmittel, um die Verbindung der Formel XIII zu erzeugen.

**14.** Verfahren nach Anspruch 13, worin jedes R ausgewählt ist aus $CH_3$, $C_2H_5$ und n-$C_3H_7$.

**15.** Verfahren nach Anspruch 13 oder 14, worin X Cl oder Br ist.

**16.** Verfahren nach Anspruch 15, worin jedes R $CH_3$ ist und X Cl ist.

**17.** Verfahren zur Herstellung einer Verbindung der Formel XV,

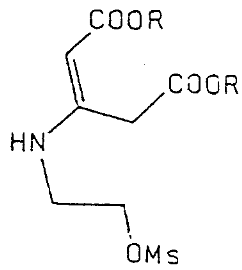

(XV)

worin
Ms Mesyl ist; und
jedes R unabhängig $C_1$-$C_6$-Alkyl ist,
umfassend das Behandeln eines Di($C_1$-$C_6$-alkyl)-1,3-acetondicarboxylats,

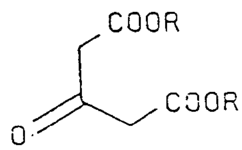

worin R wie oben definiert ist,
mit $HOCH_2CH_2NH_2$ in einem aprotischen Lösungsmittel, um ein Hydroxyenamin (XIV) zu erzeugen;

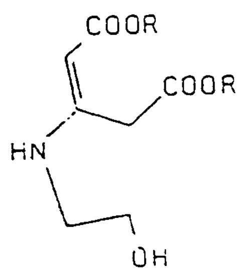

(XIV)

und
das Behandeln des Hydroxyenamins (XIV) mit Mesylchlorid und einer Base in einem aprotischen Lösungsmittel.

**18.** Verfahren nach Anspruch 17, worin jedes R ausgewählt ist aus $CH_3$, $C_2H_5$ und n-$C_3H_7$.